# EUROPEAN PATENT APPLICATION

(11) **EP 4 706 539 A1**
(43) Date of publication of application: **11.03.2026**
(21) Application number: 24198605.8
(22) Date of filing: 05.09.2024
(51) Int. Cl.: A61B 5/262, A61B 5/263

(54) **DEVICE FOR INTERFACING A BODILY TISSUE, COMPRISING AT LEAST ONE GLASSY CARBON MICRONEEDLE AND METHOD FOR PRODUCING THE DEVICE**

(71) Applicant: ECOLE POLYTECHNIQUE FEDERALE DE LAUSANNE (EPFL), 1015 Lausanne (CH)
(72) Inventor: FERNÁNDEZ LAVADO, Emilio, 1007 Lausanne (CH); AKOUISSI, Outman, 1004 Lausanne (CH); FURFARO, Ivan, 1213 Petit-Lancy (CH); LACOUR, Stephanie P., 1163 Etoy (CH)
(74) Representative: M. Zardi & Co S.A.

(57) **Abstract**

It is disclosed a device (1) for interfacing a bodily tissue comprising:
a flexible and/or elastic substrate (100), including at least a first surface (101) and a second surface (102), and at least one microneedle (200), including a body protruding from at least one of said first surface (101) and second surface (102) of the flexible and/or elastic substrate (100).

The device (1) is characterized in that the body of the at least one microneedle (200) is made of glassy carbon and is operatively coupled with at least one fluidic channel (300) and/or with at least one electrically conductive track or pad (400), running in or on the flexible and/or elastic substrate (100).

## Description

### Technical Field

The invention belongs to the field of devices for interfacing a bodily tissue. In particular, the invention relates to a soft bioelectronic interface for unidirectional or bidirectional interaction with bodily parts of a user, for instance for biomedical purposes.

### Background Art

In the field of devices for interfacing a bodily tissue, such as bioelectronic wearable systems for healthcare, there is a growing demand for reliable, non-invasive technologies capable of continuous monitoring of electrophysiological signals and precise electrical stimulation through the skin. Traditional wet skin interfaces, requiring gels or liquids to maintain low electrode impedance, often result in variable signal quality and user discomfort. Recent advancements focus on dry skin interfaces using microneedle electrodes that penetrate the epidermis, reducing skin-electrode impedance and eliminating the need for conductive gels.

Microneedle arrays offer a painless and direct way of interfacing with the skin. Skin bioelectronic interfaces based on microneedle arrays have emerged as a promising solution for developing the next generation of health wearable systems. Delivery of drugs and vaccines, biochemical sensing of health markers, electrophysiological monitoring, wound healing and transdermal stimulation of nerves are all possible applications where these interfaces could make an impact in the everyday life of many people.

However, skin bioelectronic interfaces based on microneedle arrays as presently known are affected by some problems, due to limitations of physical and electrical properties of material used for their manufacturing and for criticalities of the manufacturing process in itself.

In particular, silicon and ceramics are materials with good mechanical strength, and indeed they can effectively and reliably penetrate the stiff biological tissues (e.g. skin) thanks to their hardness. Nonetheless, they do not possess sufficient electrical conductivity so that they cannot establish an electrical interface with the tissues. On the other hand, most metals with high electrical conductivity such as platinum or gold are very ductile materials so they are not able to penetrate the skin due to mechanical bending. In any case, it is quite challenging to fabricate customizable miniaturized shapes using metal or ceramics in a scalable way due to the complexity in the microfabrication process.

As an alternative, polymers are easy to pattern in a more scalable way, for instance by means of photolithography, which simplifies the fabrication process and allows to create complex three-dimensional shapes such as hollow microneedles, for establishing a fluidic interface with biological tissues. Recent advancements in chemistry have made it possible to tune the electrical and mechanical properties of many polymers (using for example hydrogels). However, the hardness and conductivity of microneedle based on polymers are still not ideal for developing a robust and reliable interface.

With the aim of leveraging and combining the diverse properties of these materials to obtain good electromechanical properties, a common approach during the last years has been the use of conductive coatings (or cores) on top of (or inside) hard structures. The coatings or cores are often made of metal and the hard structures are made of ceramics or polymers. However, the use of several materials on the same structure limits its robustness and its functionality, while increasing the complexity of the fabrication process. For example, many polymer microneedles coated with a thin film metallic layer are known to electrically record information from the skin. However, these devices cannot be used for electrical stimulation because the metal would delaminate from the polymer due to its weak adhesion interface.

### Summary of invention

In order to address and overcome at least some of the above-mentioned drawbacks of the prior art solutions, the present inventors developed a device and a method for manufacturing the same, having improved features and capabilities.

In particular, a first purpose of the device as disclosed is that of coupling rigid microneedles into for instance a thin, flexible/elastic substrate to ameliorate and enhance physical contact compliance between the device and a user bodily parts.

A further purpose is that of implementing a manufacturing method for scalable producing a reliable device.

Still a further purpose is that of providing a device for a multimodal (electrical, fluidic or electrofluidic) bidirectional (stimulation/sensing) three-dimensional (microneedle arrays) soft bioelectronic interface.

All those aims have been accomplished with the device and method as described herein and claimed in the appended claims.

In view of the above-summarized limitations of the prior art, according to the present invention there is provided a device for interfacing a bodily tissue according to claim 1.

The device includes:
- a flexible and/or elastic substrate including at least a first surface and a second surface, in particular the second surface being opposite to the first surface and
- at least one microneedle including a body on at least one of said first surface and second surface of the flexible and/or elastic substrate.

The device is characterized in that the body of the at least one microneedle is made of glassy carbon, and the body is operatively coupled with at least one fluidic channel and/or with at least one electrically conductive track or pad, running in or on the flexible and/or elastic substrate.

Advantageously, the glassy carbon microelectrode has optimal mechanical strength for effectively and reliably penetrate stiff biological tissues, including skin of a human. It has optimal electrical conductivity for establishing an electrical connection with the tissues, if the microneedle is used as an electrode. It is sufficiently rigid to stably delimit therein a fluid path suitable to receive fluid from the fluidic channel, if the microneedle is used (also or only) as a fluid carrier. Due to the hardness of GC, brake or accidental closure of the fluid path in the microneedle are prevented.

Advantageously, the device is multimodal, meaning that it may support different modes of interaction with the bodily tissue, including an electrical mode, when an electric current is transferred through the microneedles and electric conductive track of pad, a fluidic mode, when a fluid is transferred through the microneedles and the fluidic channel, or a combined electrical and fluidic mode, when both an electrical current and a fluid is transferred through the body of the microneedles, the electrically conductive track or pad and the fluidic channel.

The modes of interactions cited above are not limiting; for instance, a heating interaction mode may be further supported by the device, to exchange heat between the device and the bodily tissue; in this respect, the body of the microneedles or the fluid inside the microneedles may form a medium to exchange heat with the bodily tissue; a sensor temperature and a processor in the device may be configured to sense temperature changes over time with respect to an initial temperature on the fluid. Based on the initial temperature and its change in time, a temperature of the bodily tissue may be measured. Moreover, heating and cooling means may increase and decrease the temperature of the medium. Accordingly, the medium may heat or cool the bodily tissue. The heating and cooling means may be integrated in the device or in an external apparatus, operatively connected to the device.

Moreover, the interaction may be mono-directional, for instance when the fluid is delivered through the fluidic channel and the microneedles from the device to the body tissue, or vice versa, from the body tissue to the device, or bidirectional, when the fluid flows in both directions (to and from the device) contemporarily. In case of bidirectional flow, a bodily fluid, for instance sweat, may flow from the body tissue to the device and another fluid, for instance a sweat promoter, may contemporarily flow from the device to the bodily tissue; the sweat may be collected into the device for analysis. The fluidic channel may be connected to a reservoir of the device, where the bodily fluid is collected.

The device is three-dimensional since the flexible and/or elastic substrate is suitable to comply to a 3D shape delimited by the body tissue part at rest or moving, therefore allowing the microneedles to implant at points of the 3D shapes arranged at different height with respect to a flat reference plane.

Although the interaction between the device and the bodily tissue may include penetration of the microneedle through the bodily tissue, the depth of penetration, the amount of fluid exchanged and the intensity of electrical current transmitted when the device is in use is up to a degree that said use has not to be read as a treatment of the human or animal body excluded from the patentability. Indeed, in use, the device implements an alternative method to known treatments that are not excluded from patentability, such as method to stimulate bodily tissues by applying an electric current through interposition of a gel between the body tissue and device terminals or method for virtual- or augmented-reality haptic application. In no one these methods or applications, the microneedle(s) cause cuts into the body tissue; in particular, microneedles insertion into and extraction from the bodily tissue do not cause any bleeding. To the contrary, the bodily tissues is substantially unaltered by the microneedles (no change may be perceived before and after insertion into the bodily tissue of the microneedles). In particular, the bodily tissue is the epidermis.

In one embodiment, at least one microneedle is arranged on both sides of the flexible and/or elastic substrate, and the body of each of the microneedles on both sides of the substrate is operatively coupled with at least one fluidic channel and/or with at least one electrically conductive track or pad, running in or on the flexible and/or elastic substrate. This embodiment may be advantageous for application of the device between two bodily portions faced one to the other, for instance two tongues.

Although reference is made to at "least one" microneedle, according to another aspect of the disclosure, a plurality of microneedles is arranged on one or both sides of the flexible and/or elastic substrate, and the body of each of the microneedle is operatively coupled with at least one fluidic channel and/or with at least one electrically conductive track or pad, running in or on the flexible and/or elastic substrate.

In one embodiment, the body of the microneedle includes a stem and a support base of the stem. A proximal end of the stem is connected to the support base, preferably monolithic therewith, and the support base is operatively connected with the flexible and/or elastic substrate of the device. The stem has a height in a Z direction, a thickness in the X and Y direction; the substrate has a height in Z direction (which is the thickness of the substrate), and extends in the X and Y direction (where it delimits the first and second surfaces). The stem is higher than a height of a portion of the substrate in which the microneedle is coupled to the substrate, in the order from tens to hundreds of times of the height of such portion of the substrate. The length of the support base may be in the order of magnitude of the height of the stem. The length is the distance of a distal end of the support base from the stem. The support base of the microneedle keeps the stem stably on the substrate. The support base of the microneedle is preferably (all or partially) around the stem.

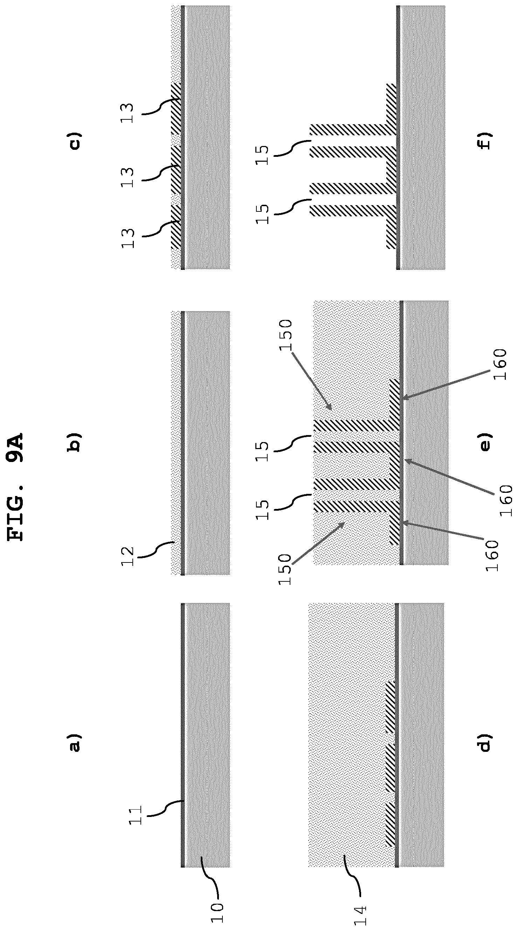

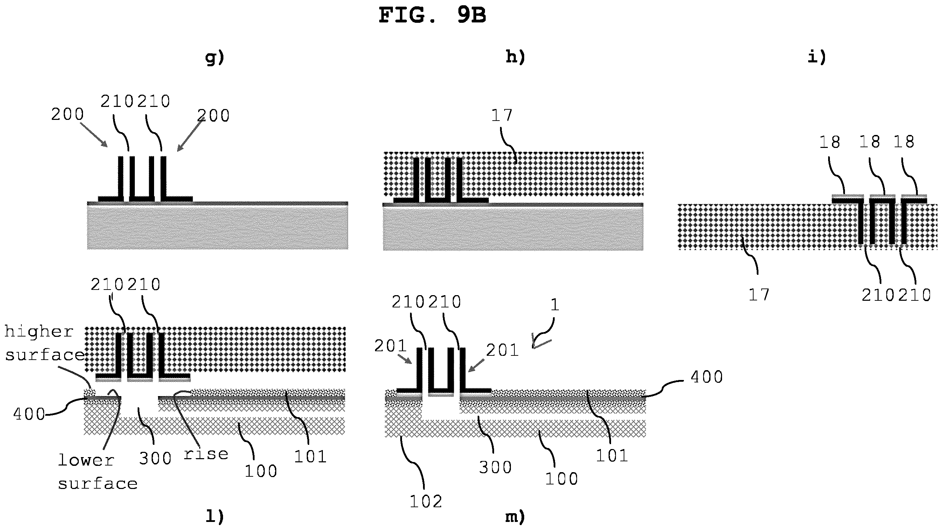

Adjacent stems may share a portion of the support base. In this case, the stems of the adjacent microneedles are distanced by means of the support base (Figure 9B-m).

A plurality of microneedles may be arranged, according to a pattern, to functionally cover an area of the flexible and/or elastic substrate. Each one of a plurality of stems of the plurality of microneedles may share one or more portions of the support base with one or more adjacent stems. The stems arranged at a periphery of the area functionally covered have at least a portion of the support base not shared with other stems. In one embodiment, the not shared support base engages the flexible and/or elastic substrate for said operative connection of the flexible and or elastic substrate with the microneedles.

For instance, the fluidic channel is delimited in the thickness of the flexible and/or elastic substate and an aperture is on a portion of the first surface of the substrate, in fluid communication with the fluidic channel. The support base of the stems which is not shared with other stems is coupled with the first surface, around the aperture thereof, to close it and allows in this way the delivery of the fluid coming from the fluidic channel towards the stems.

Advantageously, this coupling between the support base and the flexible and/or elastic substrate is very reliable because it may count on a wider surface (the one of the support bases) than the thickness of the stem. Advantageously, the support base(s) extended in parallel to the flexible and/or elastic substrate, when the substrate is at rest (not in use or plain) and offer an optimal coupling to the substrate; the stems, instead, extends perpendicularly to the support base(s).

Moreover, preferably, the first surface includes a step around the aperture. In other words, the first surface includes a lower surface forming a first tread of the step, a higher surface forming a second tread of the step and a rise connecting the lower and the higher surface of the step (Fig. 9B-I). The lower surface delimits the aperture. The lower surface extends on a plane, at rest (i.e. when the flexible and/or elastic substrate is not bent). The higher surface extends at a higher height than the aperture. In particular, at rest, the higher surface extends on a plane higher than the plane on which the lower surface extends. The support base of the stems which is not shared with other stems fits into the lower surface. An exposed surface of the support base (the one not in contact with the lower surface) may be coplanar with the higher surface. Fitting the support base into the step of the first surface (Fig. 9B-m) protects the coupling between the flexible and/or elastic substrate and the body of the microneedles.

As said above, the body is operatively coupled with the at least one fluidic channel. In one embodiment, suitable for fluidic application, the body is at least in part hollow. In particular, it is the stem to be hollow. In one embodiment, the stem is entirely hollow, i.e. it includes a through hole passing from the proximal end to the distal end of the microneedle, in the axial (Z) direction. In another embodiment, the stem is partially hollow, i.e. it includes a through hole passing from the proximal end to an intermediate portion of the stem, in the axial (Z) direction. At least an opening in a lateral wall of the stem allows the fluid to flow in and/or out the stem.

In one embodiment, the hollow body part of the microneedle (the stem) is in fluid communication with the at least one fluidic channel for operatively conduct a fluid from the fluidic channel to an aperture of the hollow body part (the stem), and the body is in electric contact with the at least one electrically conductive track or pad. For instance, the aperture is at the distal end of the microneedle and the electric contact is at the support base.

In another embodiment, the body is operatively coupled with the at least one electrically conductive track or pad, the body is in electric contact with the at least one electrically conductive track or pad, and the body is non-hollow. A non-hollow body means that the stem is not-hollow. Coupling of the microneedle with the flexible and/or elastic substate may be similar to what disclosed above. For instance, one or more support bases of the stems are electrically connected to one or more conductive tracks or pads.

In a preferred embodiment, the at least one microneedle protrudes from a glassy carbon support, monolithic with the at least one microneedle, wherein the glassy carbon support is coupled to the flexible and/or elastic substrate, preferably arranged thereon, to realize the operative coupling between the at least one microneedle and the at least one fluidic channel and/or with at least one electrically conductive track or pad.

For instance, the support base of the stem of the needle may form the glassy carbon support. In case of a plurality of microneedles, the support bases of the stems of the microneedles all together may form the glassy carbon support. The support bases form a continuous glassy carbon support board.

In one embodiment, the at least one microneedle is at least partially coated with an electrically conductive film. For instance, the support base of the microneedle is covered with electrically conductive film to improve electrical connection with tracks or pads.

The flexible and/or elastic substrate comprises a flexible printed circuit board (PCB).

It is herein summarised also the process for manufacturing the device. The process is claimed in claim 9.

The microfabrication method for manufacturing the device for interfacing a bodily tissue, comprising the steps of:
- providing a flexible and/or elastic substrate having at least a first surface and a second surface,
- providing at least one polymeric body on a carrier, said polymeric body having the shape of a needle and protruding from a proximal end on the carrier to a distal end distanced from 10 to 2000 microns from the carrier. This dimension is the reasons why the needle is also said microneedle, after manufacturing. Preferably, the polymeric body protrudes from the proximal end to the distal end from 400 to 1400 microns.

The method further comprises the steps of
- carbonizing via pyrolysis the at least one polymeric body and obtaining a corresponding at least one glassy carbon microneedle;
- detaching the at least one microneedle from the carrier;
- Transferring the at least one microneedle onto the flexible and/or elastic substrate, to operatively coupling the at least one microneedle with at least one fluidic channel and/or with at least one electrically conductive track or pad, running in or on the flexible and/or elastic substrate.

The step of detaching from the carrier preferably includes:
- inserting the at least one microneedle into a penetrable stamp, wherein detaching from the carrier is made by flipping the stamp with the at least one microneedle, and the step of transferring the at least one microneedle includes transferring the stamp with the at least one microneedle inserted therein onto the flexible and/or elastic substrate.

The stamp is then removed from the at least one microneedle after transferring.

Advantageously, a thin stem or a plurality of thin stems protruding from the carrier may be protected by the stamp in which they penetrate, so as their structural shape and reciprocal distance is safeguarded in the phases of coupling the microneedles with the flexible and/or elastic substrate. In case of support bases to be coupled around an aperture of the first surface of the substrate, the stamp allows to simplify the operative coupling, for instance precisely arranging the support base in the stepped portion of the surface.

In one embodiment, the step of providing at least one polymeric body on the carrier comprises patterning a polymeric support on the carrier and patterning the polymeric body on the polymeric support, wherein the step of carbonizing carbonizes the at least one polymeric body and the polymeric support in a monolithic glassy carbon unit. Carbonization of the polymeric support determines the support base(s) of the stem(s) and carbonization of the polymeric body determines the stem(s).

Preferably, the at least one microneedle, in particular the support base thereof, is at least partially coated with an electrically conductive film, such as a metallic film. The step of partially coating is carried before detaching the at least one microneedle from the stamp.

More preferably, the carrier comprises a thin-film oxide sacrificial release layer, and the at least one polymeric body is provided on the thin-film oxide sacrificial release layer of the carrier.

It is herein precisely disclosed the design, fabrication, and characterization of glassy carbon microneedle electrode arrays (GC-MEAs) on flexible substrates for bodily tissue electrical interfacing. In implemented and non-limiting embodiments, the fabrication process includes photolithography, pyrolysis, and transfer printing techniques. This combination of techniques enables the scalable production of large, customizable GC-MEAs with lengths ranging from 150 to 400 µm and base diameters from 50 to 150 µm on flexible polyimide printed circuit boards.

Extensive characterization tests demonstrated the suitability of glassy carbon for a reliable dry skin interface, with a hardness of 3.5 GPa and a conductivity of 103 S/m. Numerous experiments, including hematoxylin-eosin (H&E) staining and impedance spectroscopy, confirmed effective skin penetration using various explanted animal skin models. However, devices according to the present disclosure are suitable for several applications beyond skin-related ones, such as for instance as bioelectronic interfaces for physiological monitoring or stimulation of internal organs of a subject.

The successful integration of glassy carbon microneedles on flexible substrates opens up a wide range of potential applications for, for example, health wearable systems, including for instance collecting electrophysiological data (EMG, ECG, EEG) from both animals and humans using this technology, as well as on modulating the nervous system via transdermal electrical stimulation, among others.

Advantageously, the plurality of microneedles can be patterned on the glassy carbon support, thereby forming a monolithic glassy carbon unit comprising a support and an array of microneedles, said monolithic glassy carbon unit being operatively connected to the flexible and/or elastic substrate. The glassy carbon support is preferably manufactured concomitantly with the microneedles, in a process creating the glassy carbon unit in a single step.

The above and other features and advantages of the device as discloses will become more apparent from the following description given with reference to the attached drawings.

### Brief description of drawings

FIG. 1A is a schematic perspective view of an embodiment of a device as disclosed; the device includes a fluidic interface, comprising hollow glassy carbon microneedles in fluidic connection with a fluidic path in a flexible and/or elastic substrate of the device; FIG. 1B) is a schematic perspective view of the device as disclosed, according to another embodiment; the device includes an electrical interface, comprising solid glassy carbon microneedles in electrical connection with a conductive path and a pad on the substrate;
FIG. 2 is a schematic perspective view of the embodiment of FIG. 1B according to another aspect of the disclosure, wherein the microneedles are embodied as a monolithic structure together with a glassy carbon support unit, the monolithic structure being in electrical connection with the conductive path and the pad located on the substrate;
FIG. 3 is a schematic perspective view of the embodiment of FIG. 2, according to another aspect of the disclosure wherein only conductive path is provided (the pad is absent);
FIG. 4A is a schematic perspective view of an embodiment of the device as disclosed comprising hollow glassy carbon microneedles in a glassy carbon monolithic structure, the microneedles being operatively (fluidically) connected to fluidic path(s) patterned into the support substrate to create an electrofluidic interface device; FIG. 4B is a schematic perspective view of the embodiment of the device of FIG. 4A according to a variant in which only the fluidic interface is provided (electrical interface is not provided).
FIG. 5 is a schematic perspective view of an embodiment of the device as disclosed, operatively connected with both an external fluidic and an external electrical device;
FIG. 6 is a schematic perspective view of non-hollow microneedles of the device as disclosed, according to different possible embodiments;
FIG. 7 is a schematic perspective view of hollow microneedles of the device as disclosed, according to different possible embodiments;
FIG. 8 is a front view (longitudinal cross-section) of contour shapes of the microneedles of the device as disclosed, according to different possible embodiments;
FIG. 9A and 9B schematically represent steps of a manufacturing process of the device as disclosed;
Figure 9C is a detail of the device as manufactured at a last step of Figure 9B.
FIG. 10 is a diagram representing some real combination of dose energy and gap tested for the manufacturing of microneedle arrays, wherein the manufacturing includes photolithographic patterning the microneedle arrays;
FIGS. 11 to 16 show the characterization of real glassy carbon microneedles devices, namely:
   Raman spectroscopy (FIG. 11);
   EDX spectroscopy (FIG. 12);
   Mechanical tests (FIG. 13A and 13B);
   Resistivity tests (FIG. 14);
   Electrochemical impedance spectroscopy (FIG. 15A and 15B);
   Skin penetration proof (FIG. 16);
FIG. 17A to 17C shows a real fabricated device containing microneedle array electrodes as well as planar electrodes of the same material (glassy carbon) in order to compare their properties when recording electrophysiological data from animals.

### Detailed description of the invention

The device according to the present invention is in the following disclosed with reference to the drawings. The device is globally indicated with reference number 1.

It is however to be understood that the scope of protection of the invention is not limited to those aspects described in the following and represented in the drawings; to the contrary, the scope of protection of the invention is defined by the claims. Moreover, it is to be understood that the specific conditions or parameters described and/or shown in the following are not limiting of the scope of protection of the invention, and that the terminology used herein is for the purpose of describing particular aspects by way of example only and is not intended to be limiting.

Unless otherwise defined, technical and scientific terms used herein have the same meaning as commonly understood by one of ordinary skill in the art to which this invention belongs. Further, unless otherwise required by the context, singular terms shall include pluralities and plural terms shall include the singular. The methods and techniques of the present disclosure are generally performed according to conventional methods well known in the art and as described in various general and more specific references that are cited and discussed throughout the present specification unless otherwise indicated. Further, for the sake of clarity, the use of the term "about" is herein intended to encompass a variation of +/- 10% of a given value.

In the following description, an orthogonal reference frame XYZ is defined with three axes perpendicular to each other, namely:
- an X axis, defining a longitudinal, horizontal direction,
- a Y axis, defining a transverse direction with respect to the horizontal direction of the X axis, or vertical direction; X and Y axis define a vertical XY plane,
- a Z axis, defining a transverse direction with respect to the vertical XY plane, perpendicular to the vertical XY plane.

The following description will be better understood by means of the following definitions.

As used in the following and in the appended claims, the singular forms "a", "an" and "the" include plural referents unless the context clearly dictates otherwise. Also, the use of "or" means "and/or" unless stated otherwise. Similarly, "comprise", "comprises", "comprising", "include", "includes" and "including" are interchangeable and not intended to be limiting. It is to be further understood that where for the description of various embodiments use is made of the term "comprising", those skilled in the art will understand that in some specific instances, an embodiment can be alternatively described using language "consisting essentially of" or "consisting of."

In the frame of the present disclosure, the expression "operatively connected" and similar reflects a functional relationship between the several components of the device or a system among them, that is, the term means that the components are correlated in a way to perform a designated function. The "designated function" can change depending on the different components involved in the connection. A person skilled in the art would easily understand and figure out what are the designated functions of each and every component of the device or the system of the invention, as well as their correlations, on the basis of the present disclosure.

"Glassy carbon" is a bulk solid based on strongly curved sp2-bonded graphene sheets, while amorphous carbon films are disordered even on the atomic scale and have a fraction of sp3 bonds ranging from a few to almost 100%. Glassy carbon or vitreous carbon is a non-graphitizing or non-graphitizable carbon which combines glassy and ceramic properties with those of graphite. Unlike graphite, glassy carbon has a fullerene-related microstructure. This leads to a great variety of unique materials properties. Glassy carbon is an isotropic, disordered material obtained by pyrolysis of organic precursors such as cellulose, epoxies, phenolics, polyimides, etc. It is hard, glossy, and black, fractures like typical glasses, and does not graphitize easily. The density is low, implying a high porosity, but it is still very resistant to chemical attack, especially from acids and many molten metals, and has a low permeability for gases. Because it is difficult to machine, products are usually formed to their final shapes in the initial polymer stage. Carbonization by slow heating to 1300±1600K results in a weight loss and volumetric shrinkage by about 50%, and the material can then be treated at temperatures up to 2500K with little further dimensional change. Glassy carbon can also be formed as a surface coating.

Glassy carbon consists of twisted graphene-like ribbons or sheets forming a disordered network of walls between voids of typical dimensions 1-5 nm, it has a hardness of 2 to 8 GPa and a Young's modulus near 30GPa. The mechanical and electrical properties of glassy carbon greatly depend on the manufacturing parameters to fabricate it (mainly, pressure and temperature) as well as on the used precursor.

The term "flexible" is used herein to refer to elements of the device or a system according to the invention that can perform a deflection. Generally speaking, the term "deflection" refers to any displacement, expansion, contraction, bending, torsion, twist, linear or area strain, or any other kind of deformation, of at least a portion of the said device or system, for instance a polymeric structure.

Certain materials used in accordance with the present invention are soft polymers used for producing elastic supports. In the frame of the present disclosure, a "soft" material is any material that is either compressible, reversibly compressible, elastic, stretchable or any combination thereof. Accordingly, elastic supports according to the present disclosure are substantially made of (i.e., consist of) soft materials, preferably soft polymers. The term "stretchable" refers to the elastic behaviour of an item and is herein used to mean an intrinsic or engineered property of a material or structure that allows such material or structure to withstand a large elongation or multidirectional strain upon a strain stress, upon a single or multiple cycles, comprised between 1 and 500%, typically of >5% of the elongation of a soft structure at rest, such as for instance more than about 10%, more than about 20%, more than about 50%, more than about 100% or even more than about 200% of a soft structure at rest without cracking or loss of its physical and/or mechanical properties, which represents an advantage in those contexts in which several cycles of mechanical stresses over time can be foreseen.

Certain polymers composing the flexible and/or elastic substrate according to the present disclosure may comprise one or more compounds selected from a non-exhaustive list comprising thermosets or thermoplastics such as styrene butadiene, styrene (SBS) or styrene ethylene butylene styrene (SEBS), alkyds, epoxies, phenolics (e.g., Bakelite), polyimides, formaldehyde resins (e.g., urea formaldehyde or melamine formaldehyde), polyester thermosets, unsaturated polyesters, polyurethane, bismaleimides (BMI); polyvinyl chloride (PVC), neoprene, uncrosslinked neoprene, polyethylene (PE), cross-linked polyethylene (PEX), polyether, ethylene-vinyl acetate (EVA), polyethylene-vinyl acetate (PEVA), polypropylene glycol (PPG), latex; elastomeric materials such as silicone rubber (e.g. polydimethylsiloxane PDMS) or fluorosilicone rubber; thermoplastic elastomers such as styrenic block copolymer (SBC), ethylene propylene diene monomer (EDPM) rubber, butyl rubber, nitrile rubber; poly(lactic-co-glycolic acid) (PLGA), lactide and glycolide polymers, caprolactone polymers, hydroxybutyric acid, polyanhydrides, polyesters, polyphosphazenes, polyphosphoesters and poly(glycerol sebacate acrylate), polypropylene, polypropylenoxide or their derivatives, polymethylenoxide or its derivatives, polyethylene or its derivatives such as polyethylene glycole (PEG), polyethylenoxide or their derivatives, polyacrylate or its derivatives such as poly(2-hydroxyethylmethacrylate) (PHEMA), poly(vinyl alcohol) (PVA), poly(lactic acid), poly(methacrylic acid), and copolymers, poly(vinylpyrrolidone) (PVP) and combinations thereof; as well as any combination of the foregoing.

The expressions "film" or "thin film" relate to the thin form factor of an element of the device of the invention such as an electrically conductive portion. Generally speaking, a "film" or "thin film" as used herein relates to a layer of a material having a thickness much smaller than the other dimensions, e.g. at least one fifth compared to the other dimensions. Typically, a film is a solid layer having an upper surface and a bottom surface, with any suitable shape, and a thickness generally in the order of micrometers or millimetres, depending on the needs and circumstances, e.g. the manufacturing steps used to produce it. In some embodiments, films according to the invention have a thickness comprised between 0.1 µm and 5 mm, such as between 5 µm and 5 mm, between 5 µm and 1 mm, between 10 µm and 1 mm, between 5 µm and 500 µm, between 50 µm and 500 µm between, between 50 µm and 150 µm, 100 µm and 500 µm or between 200 µm and 500 µm. In some alternative or additional embodiments, according to the circumstances, films according to the invention have a thickness comprised between 5 nm to 500 nm, such as between 10 nm and 200 nm. This is the case for instance of metallic thin films used in accordance with the present invention. Higher thickness films are for instance, and without limitation, those used for manufacturing soft (flexible and/or elastic) substrates according to the invention.

A "photoresist" is a photosensitive material used in the microelectronics industry to form a patterned coating on a substrate or support surface via certain processes, such as photolithography and photoengraving. Examples of suitable photoresist usable according to the invention comprise PMMA (polymethylmethacrylate) as a positive resist and SU-8 (epoxy-based polymer) as a negative resist. These materials are typically spin-coated on a flat carrier (wafer), soft baked, exposed to UV light through a mask with the desired pattern, baked again and finally developed, as will be detailed herein below.

The expression "conductive track" refers to any film, path, stripe, strand, wire or the like which is electrically conductive in nature. Conductive tracks according to the present disclosure are used to connect and/or close an electrical circuit, and are thus usually electrical connectors or "interconnects". A conductive track is generally a metallic element that conducts an electric current toward or away from an electric circuit, but can be made of any suitable electrically conductive material, including but not limited to metals such as Au, Pt, Al, Cu and the like, as well as any alloy, oxides and/or combinations thereof; conductive polymeric materials; composite material such as polymeric materials embedding metal particles and/or metal strands or stripes, including insulating materials functionalized with electrically conductive flakes or fibers, for example carbon-filled polymers; liquid metals, including alloys or oxides thereof, such as gallium; electrically conductive inks; as well as any suitable combination thereof.

A "compliant electrode" is any structure or element able to deliver an electric current, and adapted to change its shape according to the shape change of the support it adheres to without substantially compromising mechanical or electrical performance. Examples of compliant electrodes known in the art include metal thin-films (including patterned electrodes, of out-of-plane buckled electrodes, and corrugated membranes), metal-polymer nano-composites, carbon powder, carbon grease, conductive rubbers or paints, a review of which is provided by Rosset and Shea (Applied Physics A, February 2013, Volume 110, Issue 2, pp 281-307), incorporated herein in its entirety by reference. In one embodiment, stretchable electrodes as the one described in International Patent Application WO 2004/095536, incorporated herein in its entirety by reference, can be used. Alternatively, or additionally, tubular or plain elements filled with a ionic liquid, a hydrogel or with liquid metals such as mercury or gallium, or even alloys, oxides or combinations thereof, can be used. In one embodiment, the device as disclosed includes at least one compliant electrode. As apparent in the following description, the microneedles are compliant electrodes since carried by a flexible and/or elastic substrate which is suitable to comply with the shape of a bodily part, in use, including not flat bodily parts. The substrate complying with the bodily part since it adheres thereto and assumes the same curvatures of the bodily part in different portions of the bodily part. By doing so, the substrate also allows a correct positioning of the microneedles with respect to the bodily part, for instance allowing to keep a perpendicular orientation of the microneedle with respect to an external surface of the bodily part at the different portions thereof, also when such portions have different curvatures.

The electrode or track material can be deposited using a variety of techniques including, but not limited to, printing, pad printing, screen printing, silk screening, flexography, gravure, offset lithography, inkjet, painting, spraying, soldering, bonding deposited using touch-less technologies or otherwise transferred onto the surface of a membrane. In an embodiment, the electrode can be formed by depositing an electrically conductive coating or layer by spraying a preselected onto the designated surface region. Alternatively, the electrode can be formed by depositing the electrically-conductive material onto a region of the membrane by vacuum deposition or printing the electrically conductive material on the designated surface region. This provides an electrically conductive coating of a desired thickness and a relatively uniform electrode through the desired area. Printing processes can include pad printing, screen printing and the like. Touch-free technologies such as positive material deposition of ink such as from a syringe or similar device can also be used to transfer conductive film or ink onto the membrane or substrates that are sensitive to pressure.

The device as disclosed is also said a "microfluidic device" or "microfluidic chip", meaning that materials, particularly fluid borne materials, such as liquids, can be transported through the device, in some embodiments on a micro-scale, and in some other embodiments on a nanoscale. Thus, the device as disclosed can comprise microscale features, nanoscale features, and combinations thereof.

The device may comprise structural or functional features dimensioned on the order of a millimeter-scale or less, which are capable of manipulating a fluid at a flow rate on the order of a µL/min or less. Such features include, but are not limited to channels, fluid reservoirs, reaction chambers, mixing chambers, and separation regions. In some examples, the channels include at least one cross-sectional dimension that is in a range of from about 0.1 µm to about 500 µm. The use of dimensions on this order allows the incorporation of a greater number of channels in a smaller area, and utilizes smaller volumes of fluids.

The device as disclosed can be used alone or as part of a microfluidic system which, for example and without limitation, can include: pumps for introducing fluids, e.g., samples, reagents, buffers and the like, into the system and/or through the system; detection equipment or systems; data storage systems; and control systems for controlling fluid transport and/or direction within the device, monitoring and controlling environmental conditions to which fluids in the device are subjected, e.g., temperature, current, and the like.

As used herein, the terms "channel," "micro-channel," "fluidic channel," and "microfluidic channel" are used interchangeably and can mean a recess or cavity formed in a material by imparting a pattern from a patterned substrate into a material or by any suitable material removing technique, or can mean a recess or cavity in combination with any suitable fluid-conducting structure mounted in the recess or cavity, such as a tube, capillary, or the like. In the present invention, channel size means the cross-sectional area of the microfluidic channel.

The microfluidic chip can be made from any suitable materials, such as PDMS (Polydimethylsiloxane), glass, PMMA (polymethylmethacrylate), PET (polyethylene terephthalate), PC (Polycarbonate), to cite a few, or a combination thereof.

The term "subject" as used herein refers to animals, particularly mammals. For example, mammals contemplated by the present invention include human, primates, domesticated animals such as cattle, sheep, pigs, horses, laboratory rodents and the like. The device as disclosed is for interfacing a bodily tissue of a subject.

A "fixed implant" defines a biomedical device having the ability to conform to established and/or customised surgical procedures and to reside in vivo without producing adverse biological reactions over extended periods of time, such as for instance over 7 days. Still within the meaning of the present invention, a "removable implant" defines a biomedical device having the ability to conform to established and/or customised surgical procedures and to reside in vivo for a limited amount of time, such as for instance the time of a surgical operation. The device as disclosed may be a fixed implant or a removable implant.

A "bioactive agent", as well as "bioactive molecule", "bioactive compound", or "therapeutic agent", is any active agent that is biologically active, i.e. having an effect upon a living organism, tissue, or cell. The expression is used herein to refer to a compound or entity that alters, inhibits, activates, or otherwise affects biological or chemical events. Bioactive compounds according to the present disclosure can be small molecules or macromolecules, including recombinant ones. One skilled in the art will appreciate that a variety of bioactive compounds can be used depending upon the needs, e.g. a condition to be treated when the device of the invention is intended for prophylactic, therapeutic or even diagnostic purposes. A non-exhaustive list of suitable bioactive agents includes pharmacologically active substances, drugs such as antibiotics or chemotherapeutics, peptides, enzymes, antibodies, vitamins and the like.

As used herein, "treatment", "treating" and the like generally mean obtaining a desired pharmacological and/or physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" or "treating" as used herein covers any treatment of a disease in a mammal, particularly a human, and includes: (a) inhibiting the disease, i.e., arresting its development; or (b) relieving the disease, i.e., causing regression of the disease and/or its symptoms or conditions such as improvement or remediation of damage. For example, treatment of disorders of the Central Nervous System (CNS) comprises, depending on the disorder at issue, to normalize or improve somatic symptoms such as seizures, tremors, chronical infections, dementia, memory loss, intracranial pressure elevation, headache, pain, loss of feeling, loss of muscle strength, hallucinations, increased reflexes, spasticity, slurred speech, tics, paralysis, mood alterations including for instance depression, mania, euphoria and the like, phobias and so forth. As used herein, the term "prevention" or "preventing" relates to hampering, blocking or avoid a disease from occurring in a subject which may be, for any reason, predisposed to the disease but has not yet been diagnosed as having it for example based on familial history, health status or age.

The terms "diagnosing", "diagnostic" and the like as used herein refer to assessing whether a subject suffers from a disease or not. The term includes individual diagnosis the disease, or its symptoms, as well as continuous monitoring of a patient. Monitoring, i.e. diagnosing the presence or absence of a disease or the symptoms accompanying it at various time points, includes monitoring of subjects known to suffer from a disease as well as monitoring of subjects known to be at risk of developing a disease. Furthermore, monitoring can also be used to determine whether a subject is treated successfully or whether at least symptoms of a disease can be ameliorated over time by a certain therapy. Diagnosing as used herein also refers to diagnosing a predisposition of a disease and, thus, predicting whether a subject is at increased risk of developing a disease within a predictive window starting from the time when the sample to be analysed has been taken. Preferably, the predictive window is at least three months, six months, one year, two years, five years, ten years or up to the entire life span of the subject. A subject is at increased risk if the probability by which it will develop the disease is statistically significantly increased with respect to the average or mean probability, i.e. the prevalence for the disease in the respective population from which analysed subject originates.

A "narrower first end", when referred to a longitudinal section of a three-dimensional pillar or microneedle, is a relative term that is to be compared to "a wider, opposed second end" of the same longitudinal section of the three-dimensional pillar or microneedle. In the frame of the present disclosure, the first end of the longitudinal section of the three-dimensional pillar or microneedle is "narrower" compared to the second end when it comprises an edge defining a narrower angle compared to an angle located at the second end, or when it comprises a side that is smaller compared to a side located at the second end, or when it comprises a radius of curvature that is smaller compared to a radius of curvature located at the second end, as well as combinations of the aforementioned. For instance, in some embodiments, the narrower, first end of the longitudinal section may comprise a sharp or rounded edge whereas the opposed wider second end may comprise a straight side; in other embodiments, the narrower first end of a longitudinal section may comprise the sharp edge whereas the opposed wider second end may comprise the rounded edge, wherein the radius of curvature of the rounded edge is d/2.

The term pillar is used as a synonym of stem. The microneedle includes a stem, and the stem has a three-dimensional shape. The stem may have the first end, or distal end, which is narrower than the second opposed end. The second end may be coupled or forms in itself a support base of the microneedle. The support base supports the stem. In one embodiment, the support base is at least in part around the stem and extends in a direction forming an angle of inclination with the stem between 60° and 90° at the second end of the stem.

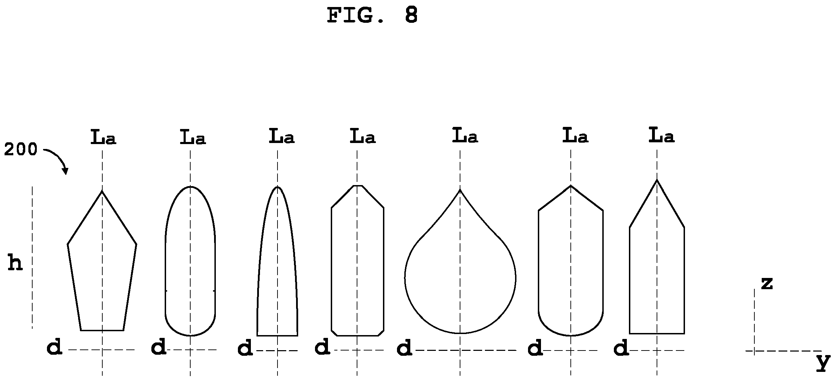

According to the above, embodiments of suitable longitudinal sections for pillars or microneedles may include triangles, trapezoids, rectangles, cones, teardrops with or without a sharp edge ("frusto-teardrop"), egglike shapes, pear-like shapes as well as regular convex polygons having an odd number of edges and irregular convex polygons having an even number of edges. Some exemplary embodiments are schematically depicted in Figure 8. Also, within the scope of the present disclosure, and in view of the above, the three-dimensional shapes of the pillar or microneedle may include without limitations any kind of pyramids, cylinders, cones, frusto-cones, parallelepipeds and the like. The pillar (stem) represented in Figure 8 has, in some embodiments, a second end narrower than the first end. In any case the pillar may be associated to support base supporting the pillar at the second end (not represented in Figure 8). In some embodiments, the support base is not provided and the second end is directly coupled to the flexible and/or elastic substrate.

Bioelectronic interfaces are the technologies that connect biological systems, such as human tissues or cells, with electronic devices. In particular, soft bioelectronic interfaces are flexible, stretchable, and/or conformable electronic devices designed to interact seamlessly with biological tissues, which are themselves soft and dynamic. These interfaces are made from materials that can bend, stretch, and conform to the contours of biological surfaces, ensuring intimate and stable contact without causing damage or discomfort. The device as disclosed is a bioelectronic interface.

The directionality of bioelectronic interfaces refers to the flow of information or signals between the biological and electronic components. They are called bi-directional if the flow of signals goes in both directions. They can convert biological signals into electronic signals (e.g.; detect the concentration of a biomarker by reading a measured voltage) and electronic signals into biological signals (e.g.: modulate a neural circuit by injecting electrical current). The device as disclosed may be bidirectional.

The modality of a bioelectronic interface refers to the method or mechanism through which the interface interacts with and exchanges information between biological systems and electronic devices. This mechanism can be electrical, optical, thermal, mechanical or chemical. They are called multimodal if the interface is able to employ several modalities at the same time. The device as disclosed may be multimodal.

The dimensionality of a bioelectronic interface refers to the spatial and structural aspects of how the interface interacts with biological tissues or cells. This can range from simple, single-point interactions to complex, multi-dimensional arrays that cover a larger surface area or volume. The dimensionality can be broadly categorized into one-dimensional (1D), two-dimensional (2D), and three-dimensional (3D) interfaces. Although three-dimensional interfaces are more invasive because they penetrate the biological system, they offer high signal resolution and selectivity, which makes the conversion of the signals much more efficient. The device as disclosed is a three-dimensional (3D) interface.

Microneedles are a type of three-dimensional bioelectronic interfaces that consist of high-aspect-ratio miniaturized (sub-mm length) hard structures that can interface with tissues (most frequently, the skin) in a minimally-invasive way (painless, for the case of the skin) by penetrating only the outermost layer of the tissue (epidermis, for the case of the skin).These microneedles should be hard (enough to penetrate the skin), conductive (enough to interface electrically with the skin) and hollow (enough to interface fluidically with the skin). Glassy carbon as disclosed in the present application is an ideal material for developing these interfaces due to its high hardness, wide electrochemical stability window, and ease of patternability through the carbonization of polymer precursors like SU-8 photoresist.

Glassy carbon is a suitable material for these interfaces due to its high hardness, wide electrochemical stability window, and ease of patternability through the carbonization of polymer precursors like SU-8 polymer.

In the following, a simple and elegant solution is disclosed which allows to tackle and overcome the shortcomings affecting known devices and manufacturing approaches in the above field of endeavor. The method solves the technical problem at the base of the invention and brings to the fabrication of a multimodal (fluidic, electrical or electrofluidic) bidirectional (stimulation/sensing) three-dimensional (microneedle arrays) soft bioelectronic interface device. Although the size and dimensionality of microneedles makes their manufacturing process very complex, the choice of material of the microneedles and the way in which these materials are treated in the manufacturing process as disclosed allows to overcome the mentioned problems.

According to the present invention, glassy carbon (GC) is used for the microneedle arrays only using this material in bulk but effectively obtaining all the desirable properties of material (such as ceramics, metals and polymers) known from prior art, while escaping from negative side effects of when such known materials are adopted.

High Patternability is achieved, since GC is obtained from a polymer precursor.

High Hardness is achieved, since GC is very hard (thus its name "glassy") as ceramics.

High Conductivity is achieved, since GC is conductive enough to constitute an electrode as metals.

The properties of this material are very suitable to create hard conductive (hollow) microneedles arrays. Moreover, glassy carbon has also proven to be chemically inert, electrochemically stable and biocompatible, which are ideal properties for any material forming a bioelectronic interface.

An important step during the fabrication is the integration of the glassy carbon microneedles in a soft substrate. This is especially hard to do in the case of microneedle arrays not only due to the mechanical mismatch between substrate (soft) and microneedles (hard) but also due to the extremely different form factor between substrate (thin film) and microneedles (high aspect ratio structures).

Additionally, it is important to take into account that glassy carbon is obtained through the carbonization at very high temperatures (> 800°C) of polymer precursors. This means that many of the techniques used in the prior art for integration the substrate with the microneedle are not compatible with the fabrication of glassy carbon. For solving this technical problem, the present inventors developed a new fabrication technique (transfer stamping) that consisted on the release of the GC 3D-microstructures from an original carrier wafer, the transfer of the GC 3D-microstructures via a stamp, and the connection of the GC 3D-microstructures with conductive tracks on a soft substrate.

Hereafter, the device 1 is disclosed with reference to Figure 9B-m.

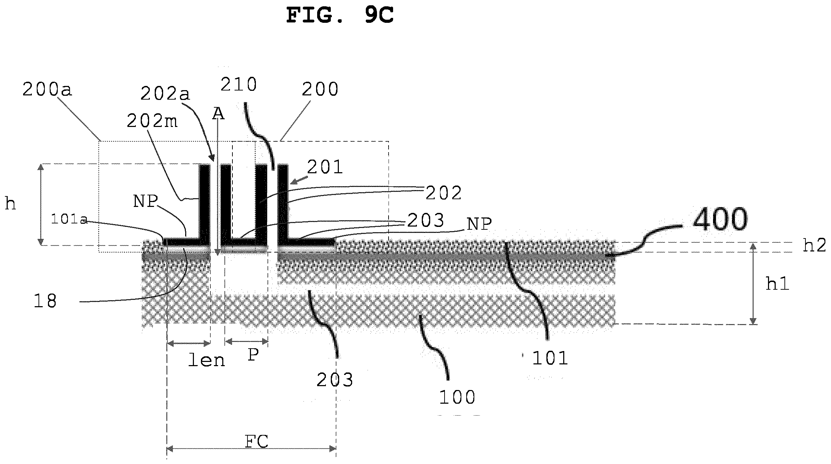

Figure 9A and 9B relate to the steps of the method for manufacturing the device 1, anFigure 9B-m, the last steps, represents schematically the device as manufactured, in a cross-section view. For clarity, Figure 9B-m is enlarged in Figure 9C. The device 1 includes a flexible and/or elastic substrate 100 including a first surface 101 and a second surface 102 and two microneedles 200, each including a body 201 on the first surface 101. The body 201 of the microneedles 200 are made of glassy carbon, and the body 201 thereof is operatively coupled with at least one fluidic channel 300 and/or with at least one electrically conductive track 400 or pad, running in or on the flexible and/or elastic substrate 100.

The body 201 of the microneedle 200 includes a stem 202 and a support base 203 of the stem 202. A proximal end of the stem 202 is connected to the support base 203, preferably monolithic therewith, and the support base 203 is operatively connected with the flexible and/or elastic substrate 100 of the device 1. The stem 202 has a height h in a Z direction, a thickens in the X and Y direction; the substrate has a height h1 in Z direction (which is the thickness of the substrate), and extends in the X and Y direction. The stem 202 is higher than a height h2 of a portion of the substrate 100 in which the microneedle 200 is coupled to the substrate 100, in the order from tens to hundreds of times of the height of the substrate. The length "len" of the support base 203 may be in the order of magnitude of the height h of the stem 202 and/or in the order of magnitude of the height h1 of the support 100. The length "len" is the distance of a distal end of the support base 203 from the stem 202. The support base 203 of the microneedle 200 keeps the stem 202 stably on the substrate 100. The support base 203 of the microneedle 200 is preferably (all) around the stem 202. Adjacent stems 200, 200a may share a portion P of the support base 203. In this case, the stems 202 of the adjacent needles are distanced from the support base 203.

A plurality of microneedles 200, 200a may be arranged according to a pattern, to functionally cover an area FC of the flexible and/or elastic substrate 100. Each one of the plurality of stems 202 of the plurality of microneedles 200, 200a may share one or more portions P of the support base 203 with one or more adjacent stems 200a. The stems 202 arranged at a periphery of the functionally covered area FC have at least a portion NP of the support base 203 not shared with other stems 202. In the embodiment of Figure 9C, both the stems 200, 200a are arranged at the periphery of the functionally covered area FC (since only two adjacent microneedles 200 are present) and each have the not shared portion NP. The not shared portion NP of the support base 203 engages the flexible and/or elastic substrate 100. The fluidic channel 300 is delimited in the thickness h1 of flexible and/or elastic substate 100 and an aperture A is on a portion of the first surface 101 of the substrate, in fluid communication with the fluidic channel 300. The portion NP of the support base 203 of the stems 202 which is not shared with other stems is coupled with the first surface 101 around the aperture A to close it and deliver accordingly the fluid coming from the fluidic channel 300 towards the stems 202. The first surface 101 includes preferably a stepped surface 101a around the aperture A and said portion NP of support base 203 of the stems 200 which is not shared fits into the stepped surface 101a so as an exposed surface of the support portion 203 (the one not in contact with the first surface) is flush with the first surface 101.

The body 201 of the microneedles of Figure 9C is at least in part hollow. In particular, the stem 202 is hollow. In one embodiment, the stem 202 is entirely hollow, i.e. it includes a through hole passing from the proximal end to the distal end of the microneedle (stem), in the axial (Z) direction. In another embodiment, the stem 202 is partially hollow, i.e. it includes a through hole passing from the proximal end to an intermediate portion 202m of the stem thereof, in the axial (Z) direction.

In one embodiment, the hollow stem 202 is in fluid communication with the at least one fluidic channel 300 for operatively conduct a fluid from the fluidic channel 300 to the aperture 202a the stem 200, and the body 201 is in electric contact with the electrically conductive track 400. For instance, the aperture 202a is at the distal end of the stem 202 and the electric contact is at the support base 203.

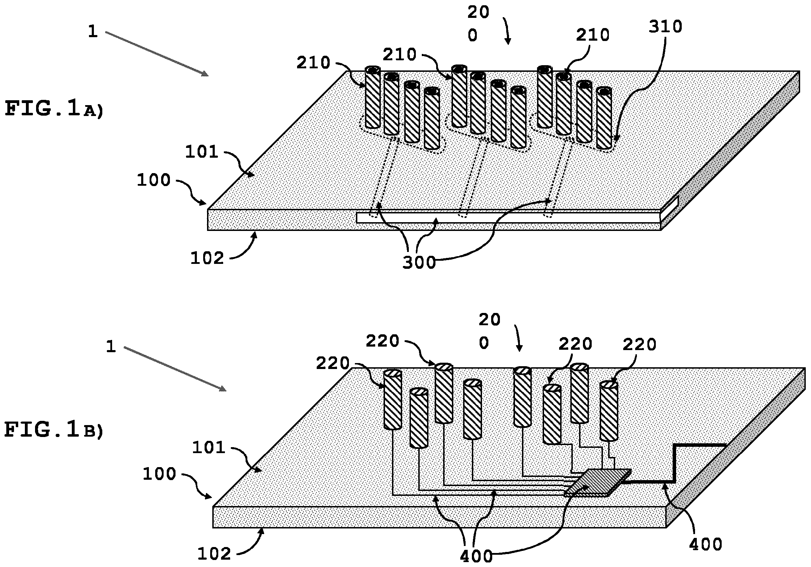

In another embodiment, the body 201 is operatively coupled with the at least one electrically conductive track or pad 400, the body 201 is in electric contact with the conductive track 400 or pad, and the body is non-hollow, such as in Figure 1B. A non-hollow body means that the stem 202 is not-hollow. Coupling of the microneedle 200 with the flexible and/or elastic substate 100 may be similar to what disclosed above. For instance, one or more support base 203 of the stems 200 are electrically connected to one or more conductive tracks 400 or pads.

The microneedle 200 may be at least partially coated with an electrically conductive film 18, in particular on the support base 203 (Figure 9C-m).

As said, Figure 9a to 9C represents the steps of the method. Thes steps are summarized as follows, before digging into more details further below.

The microfabrication method for manufacturing the device for interfacing a bodily tissue, comprise the following steps.

Providing a flexible and/or elastic substrate 100 having at least a first surface 101 and a second surface 102.

Providing at least one carrier 10 (Figure 9a).

Providing a polymeric body 150 (Figure 9e) on the carrier 10, said polymeric body having the shape of a needle and protruding from a proximal end on the carrier 10 to a distal end distanced from 10 to 2000 microns from the carrier 10.

The method further comprises the steps of
- carbonizing via pyrolysis the at least one polymeric body 150 and obtaining a corresponding at least one glassy carbon microneedle 200 (Figure 9g);
- detaching the at least one microneedle from the carrier 10 (Figure 9i);
- transferring the at least one microneedle 200 onto the flexible and/or elastic substrate 100, to operatively coupling the at least one microneedle with at least one fluidic channel and/or with at least one electrically conductive track or pad, running in or on the flexible and/or elastic substrate (Figure 9I).

The step of detaching from the carrier includes inserting the at least one microneedle 200 into a penetrable stamp 17 (Figure 9h), wherein detaching from the carrier 10 is made by flipping the stamp 17 with the at least one microneedle 200, and the step e) of transferring the at least one microneedle 200 includes transferring the stamp 17 with the at least one microneedle 200 inserted therein onto the flexible and/or elastic substrate 100 (Figure 9I).

The stamp is then removed from the at least one microneedle after transferring (Figure 9m), to obtain the final device.

In one embodiment, the step of providing at least one polymeric body 150 on the carrier 10 comprises patterning a polymeric support 160 on the carrier 10 and patterning the polymeric body 150 on the polymeric support 160, wherein the step of carbonizing carbonizes the at least one polymeric body 150 and the polymeric support 160 in a monolithic glassy carbon unit. Carbonization of the polymeric support 160 determines the support bases 203 of the stem(s) and carbonization of the polymeric body 150 determines the stem(s) 202.

Preferably, the at least one microneedle, in particular the support base 203 thereof, is at least partially coating with an electrically conductive film 18 (Figure 9i, such as a metallic film. Moreover, preferably, the carrier 10 comprises a thin-film oxide sacrificial release layer 11, and the at least one polymeric body 150 is provided on the thin-film oxide sacrificial release layer 11 of the carrier.

According to an aspect of the device as disclosed, therefore, the microneedles may be:
1) entirely hollow microneedles operatively connected with at least one fluidic channel 300 running inside the flexible and/or elastic substrate 100,
2) solid microneedles operatively connected with at least one electrically conductive track or pad 400 located in and/or on the flexible and/or elastic substrate 100 and
3) entirely hollow microneedles operatively connected with at least one fluidic channel 300 running inside the flexible and/or elastic substrate 100 and operatively connected with at least one electrically conductive track or pad 400 located in and/or on the flexible and/or elastic substrate 100.

In Figure 1a and 1b the microneedles 200 are also indicated with reference numbers 210 (Figure 1a), to indicated hollow microneedles, and 220 (Figure 1b), to indicate non-hollow microneedles.

Concerning the flexible and/or elastic substrate 100, this can comprise or be substantially composed of soft materials. Preferred soft materials may be elastomeric materials, thermoplastic elastomers (a class of copolymers or a physical mix of polymers, usually a plastic and a rubber, which consist of materials with both thermoplastic and elastomeric properties), foams, gels or hydrogels. As a way of example, the substrate 100 can be substantially composed of one or more polymers selected from a non-exhaustive and non-limiting list comprising textile materials such as cotton, silk and the like; thermosets or thermoplastics such as styrene butadiene, styrene (SBS) or styrene ethylene butylene styrene (SEBS), styrenic block copolymers, alkyds, epoxies, phenolics (e.g., Bakelite), polyimides, formaldehyde resins (e.g., urea formaldehyde or melamine formaldehyde), polyester thermosets, unsaturated polyesters, polyurethane, bismaleimides (BMI); polyvinyl chloride (PVC), neoprene, uncrosslinked neoprene, polyethylene (PE) or its derivatives such as polyethylene glycole (PEG), cross-linked polyethylene (PEX), polyether, ethylene-vinyl acetate (EVA), polyethylene-vinyl acetate (PEVA), polypropylene glycol (PPG), latex; elastomeric materials such as silicone rubber (e.g. polydimethylsiloxane PDMS) or fluorosilicone rubber, and any combination thereof; thermoplastic elastomers such as styrenic block copolymer (SBC), ethylene propylene diene monomer (EDPM) rubber, butyl rubber, nitrile rubber; poly(lactic-co-glycolic acid) (PLGA), lactide and glycolide polymers, caprolactone polymers, hydroxybutyric acid, polyanhydrides, polyesters, polyphosphazenes, polyphosphoesters and poly(glycerol sebacate acrylate), polypropylene, polypropylenoxide or their derivatives, polymethylenoxide or its derivatives, polyethylene or its derivatives such as polyethylene glycole (PEG), polyethylenoxide or their derivatives, polyacrylate or its derivatives such as poly(2-hydroxyethylmethacrylate) (PHEMA) or polyhydroxymethylacrylate, poly(vinyl alcohol) (PVA), poly(lactic acid), poly(methacrylic acid), and copolymers, poly(vinylpyrrolidone) (PVP) and combinations thereof; as well as any combination of the foregoing. These materials can be mixed with particle such as carbon nanotube, gold particles, platinum particles or diamond particle or any combinations such as to improve mechanical, electrical or thermal properties, if needed.

In cases where an elastic substrate 100 is used, the elastic behavior is provided by the materials the substrate is substantially composed of. In this context, in preferred embodiments the support substrate 100 is substantially made of a soft polymeric material, or combinations of many soft polymeric materials, particularly biocompatible ones, or polymeric materials coated with soft polymeric material or hydrogels, or made of composite materials.

In embodiments, the substrate 100 is reversibly (elastically) stretchable. In particular, a stretchable substrate 100 can withstand an elongation or multidirectional strain, upon a single or multiple cycles, comprised between 1 and 500%, preferably at least 5%, such as about 50%, about 100% or about 200%, of its size at rest without cracking or loss of its mechanical properties. Further, the substrate 100 has a Young's modulus comprised between about 1 kPa and 1 GPa, such as for instance between about 100 kPa to about 1 GPa, between about 100 kPa to about 1 GPa, between about 5 MPa to about 1 GPa, between about 100 kPa to about 100 MPa, between about 100 kPa to about 5 MPa, between about 10 kPa to about 300 kPa or between about 10 kPa to about 10 MPa, preferably between about 1 MPa to about 10 MPa, which are suitable ranges of values matching the Young's modulus of many biological tissues and surfaces to avoid mechanical mismatches between said tissues and a biomedical device, and/or for mimicking physical and/or mechanical properties of bodily tissues. In the frame of the present invention, "physical and/or mechanical properties" means, by way of examples, stress-strain behaviour, elastic modulus, fracture strain, conform ability to curvilinear surfaces, thickness, area and shape which have to be as similar as possible to those to be found in tissues of a subject's body.

In a most preferred embodiment for elastic substrates, the substrate 100 is an elastomeric substrate, for instance a PDMS substrate. The substrate 100 is preferably implemented as a thin film substrate having a flat appearance; a PDMS substrate having a thin film thickness and form factor presents several advantages in the frame of devices according to the invention, such as biocompatibility, mechanical compliance with regards to body tissue, possibility to be sterilized and high stretchability. All these features make of a PDMS thin film a particularly advantageous choice in the context of, for instance, an implantable biomedical device or wearable devices in general.

The substrate 100 is preferably, but not exclusively, implemented as a thin film of flexible and/or elastic materials, having typically a thickness comprised between 10 and 2000 mm, such as for instance between 100 and 500 mm. A substrate 100 can be composed also of a plurality of thin films stacked one on the other and bound together using common techniques such as bonding, glueing, sintering and the like. This embodiment is particularly advantageous when different materials are supposed to be used in different films to, e.g., include certain functionalities. As a way of example, a thin film composed of a flexible material such as polyimide - typically used in flexible PCBs - can be patterned and functionalized with electrical or electronic components such as electrically conductive tracks or pads and through vias, and an elastic thin film composed for instance of silicone-derived materials (e.g. PDMS) can be patterned with microfluidic channels through methods commonly known in the art and subsequently stacked below or on top of the first polyimide film, thereby creating a hybrid flexible/elastic substrate 100 with a plurality of functionalities, more easily created in the single films independently. Of note, microfluidic chips manufactured in e.g. polyimide substrates, and including electrical functionalities, are also envisageable and have been implemented in the past, as described for instance in EP3592695A1. Microfluidic chips manufactured in stretchable substrates such as PDMS, and including electrical functionalities, are also envisageable and have been implemented in the past.

According to an embodiment, the device 1 of the present disclosure comprises one or more arrays of elastically stretchable microelectrodes. As used herein, for the sake of clarity and conciseness, a "stretchable microelectrode array" refers to the ensemble of a plurality of interconnects, tracks or pads 400 and respective distal electrodes having the ability to withstand mechanical deformations such as flexing, stretching, torsion or the like, without electrical failure or loss of their electrical features. Stretchable arrays (for instance stretchable gold and/or Cr/Au microelectrode arrays, MEAs) are becoming more and more popular and find convenient applications in the field of wearable electronics, and/or biomedical interface applications, and/or even for sensing robotic skins or the like. Accordingly, microelectrode arrays are particularly suitable to be used as a neural interface with the spinal cord, brain or peripheral nerves or soft biological tissue, for instance for the purpose of stimulating and/or recording neurological or cardiac activity, as well as for monitoring hippocampal electrical activity after traumatic brain injury or bladder afferent activity, or even for stimulating electrical potential of excitable cells or the like. The device according to the present invention is suitable for all these applications, depending on the needs and circumstances. Elastically stretchable conductive tracks can be patterned on stretchable substrates such as PDMS as extensively described in the past by the present inventors, for instance in WO 2016/110564 or WO 2013/186693.

In one particular embodiment, the flexible and/or elastic substrate 100 comprises a flexible printed circuit board (PCB). This non-limiting embodiment regarding devices of the invention including flexible substrates 100 is advantageous in that it couples the flexible nature of the substrate 100 with the features and the design of printed circuit boards such as electrical tracks and/or pads, through vias, electronic components placement and the like, as well as the possibility of exploiting the valuable scalability, know-how, standardization and automation of PCB manufacturing.

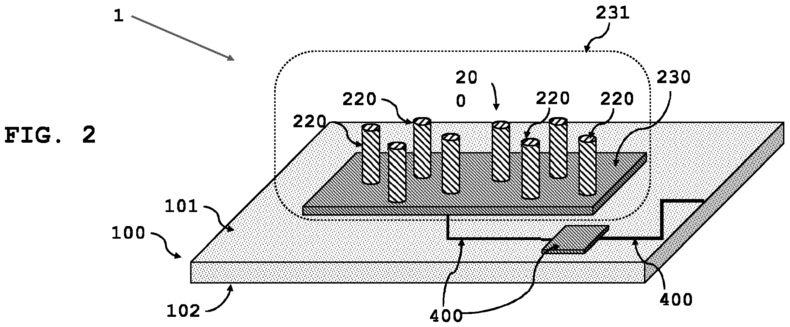

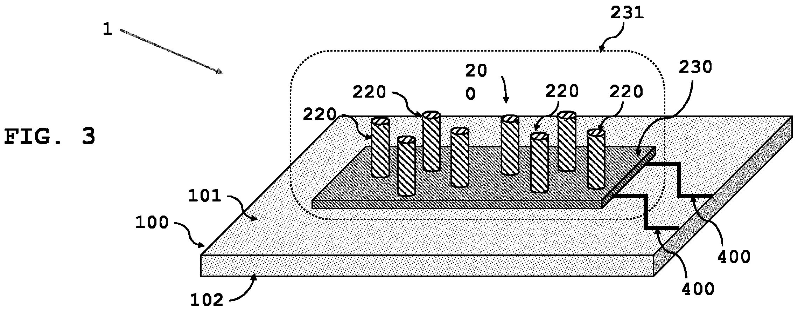

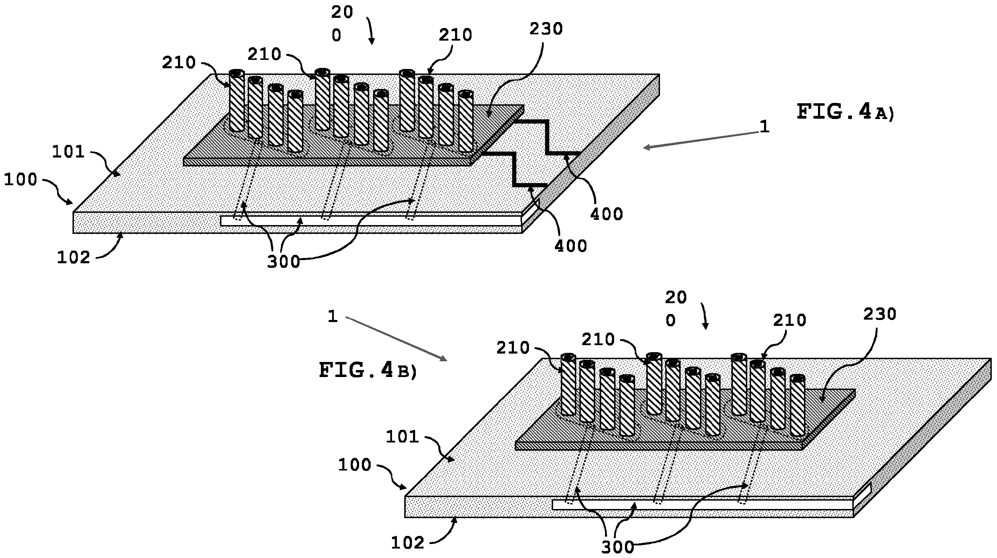

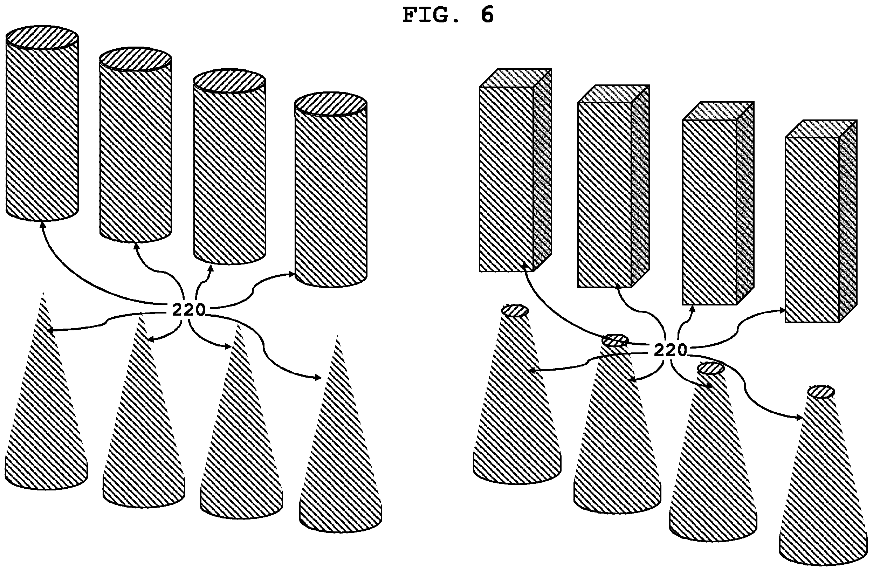

With regards to the microneedles 200, these are typically embodied as protrusions stemming from a first surface 101 of the flexible and/or elastic substrate 100 and with the narrower end or tip, whenever present, directed outwardly compared to the substrate, and they have a high aspect ratio (Figures 1 to 7). As used herein, "aspect ratio" has the meaning of the ratio between the height and the lateral feature size. This aspect of the invention is tightly linked, as will be detailed later on, to the need of the microneedles 200 to penetrate inside bodily surfaces of a subject, such as for instance the skin or internal organs such as the heart or the brain. Accordingly, in the frame of the present disclosure, for a "high aspect ratio" is intended that the ratio between the height h and the lateral sizes a (depth) or d (width) of each microneedle 200 is preferably comprised between 2 and 10 such as for instance 3, 4, 6 etc. In some embodiments, the depth a and the width d of the microneedle 200 are equal, which is typically the case of some parallelepiped, polygonal or cylindrical microneedles, and can be measured at the basis or at the top of said microneedles. The dimensions h, a, d and g (the pitch or gap distance between the various microneedles) of the microneedles 200 can usually span from 50 and 750 µm, such as for instance h being in a range from 250 µm to 750 µm; a and/or d: from 50 µm to 150 µm; g: from 100 µm to 500 µm. The microneedles 200 can be arranged in any suitable kind of array or pattern, such as square or triangular/hexagonal pattern. All the above ranges and parameters have been accurately defined by the present inventors based on the properties of the device to be achieved, the intended use, the materials and the like. For instance, the higher the aspect ratio of the microneedles, the better for penetration purposes but the weaker the microneedle might be. Therefore, if the device is intended for penetration into a softer target tissue, such as for instance a brain tissue, a higher microneedles aspect ratio might be suitable, compared for instance to penetration of skin. These considerations are important for intended use, as a way of example, but do not impact the general inventive concept behind the invention. A skilled person could easily envisage the suitable features and parameters of the needed microneedles, based on the needs, without undue burden.

In one embodiment, the microneedles 200 can be entirely hollow microneedles 210 operatively connected with at least one fluidic channel 300 running inside the flexible and/or elastic substrate 100. The expression "entirely hollow" defines microneedles or pillars of the invention comprising an inner fluidic channel spanning the entire length of the microneedles or pillars, that is, the microneedles or pillars are void along their entire height running along their longitudinal axis. In this embodiment, the device of the invention is adapted and configured to establish at least a fluidic connection between the fluidic paths comprised within the device and the subject that will be using the same. The device is therefore named, in the frame of the invention, a "fluidic interface device" or similar.

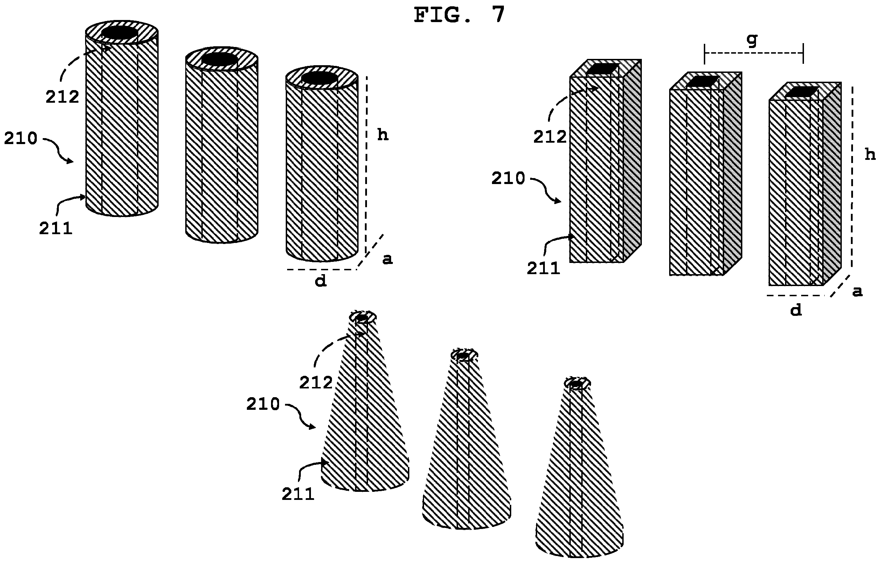

Entirely hollow microneedles 210 comprise a solid glassy carbon portion 211 (Figure 7), that may be embodied as a solid wall or more than one wall, depending on the three-dimensional shape of the microneedle (for example cylinder vs parallelepiped), and an inner void portion 212 running through the glassy carbon body. In this embodiment, the substrate 100 is patterned with methods known in the art, such as for instance by photolithography, to create fluidic paths 300 in the substrate 100, those paths 300 being fluidically and physically connected to the void portion 212 of the microneedles. The microneedles 210 can be also fluidically linked between them and to the fluidic paths 300 via intermediate units, such as for example reservoirs 310. Hundreds of possible designs are possible, that will not be described herein for conciseness reasons.

In another embodiment, the device of the invention comprises solid microneedles 220 operatively connected with at least one electrically conductive track or pad 400 located in and/or on the flexible and/or elastic substrate 100. The device is therefore named, in the frame of the invention, a "electrical interface device" or similar. As it will be evident, devices comprising combinations of solid microneedles 220 operatively connected with at least one electrically conductive track or pad 400, and entirely hollow microneedles 210 as described above, are envisageable.

In another embodiment, the device of the invention comprises a plurality of entirely hollow microneedles 210 operatively connected with at least one fluidic channel 300 running inside the substrate 100 and operatively connected at the same time with at least one electrically conductive track or pad 400 located in and/or on the flexible and/or elastic substrate 100. The device is therefore named, in the frame of the invention, a "electrofluidic interface device" or similar. The possibility of patterning glassy carbon hollow microneedles, that are inherently electrically conductive and relatively easy to pattern with a void channel in their inside, represents the main added value of the device of the present invention.

In an advantageous embodiment, the plurality of microneedles 200 is patterned on a glassy carbon support 230, thereby forming a monolithic glassy carbon unit 231 comprising a support and an array of microneedles, said monolithic glassy carbon unit 231 being operatively connected to the flexible and/or elastic substrate 100.

This embodiment has the advantage of facilitating the handling and augmenting the solidity of glassy carbon microneedles 200, during and after the manufacturing process. In fact, a glassy carbon unit 231 is much easier to manipulate as a monolithic, solid glassy carbon block that the single, tiny glassy carbon microneedles, it can represent a single contact point for electrically connecting all the microneedles to e.g. a power supply or external device 500, as well as to other electrical/electronic components of the device (for example, contact pads and the like). During the manufacturing process, as will be detailed later on along the present disclosure, a glassy carbon unit 231 facilitate the control and positioning of the microneedles in or on the flexible/elastic substrate 100 in a single step.

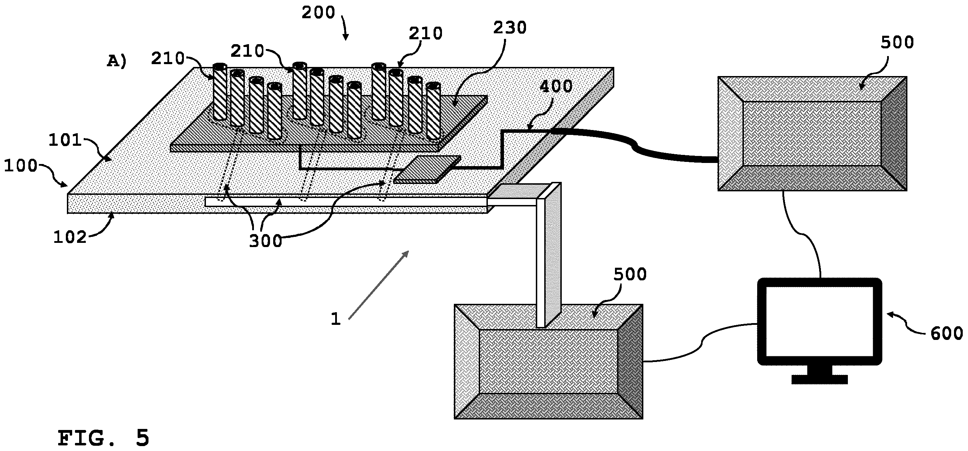

In one aspect the invention pertains to a system comprising the device as described herein operatively connected with an external device 500 through the at least one conductive track 400. For instance, the system can comprise a device as described herein operatively connected with an external device 500 selected from a non-limiting list comprising an electrostimulator, an electrical amplification and recording system, a telemetric device, a syringe pump, a pressure based pumping system and the like, as exemplarily and schematically shown in Figure 5, where two external devices 500 are operatively connected to fluidic and electrical components of an embodiment of electrofluidic device of the invention (a single external device 500 operatively connected to electrical or fluidic components of an electrical or fluidic device of the invention is of course envisageable). The external device 500 can operate in closed-loop or open-loop mode with the electric, fluidic and/or electrofluidic device of the invention. In embodiments, the device of the invention could be connected to any other "smart" device to create a system, such as a smartwatch or smartphone for example. As a way of example, for instance for virtual touch applications, e-textile gloves may be thought at, wherein the device is embedded in the body of the glove and the microneedles get in touch to the fingertips and/or palm hand of a user. Those gloves could then be connected to a smartwatch in the wrist for power and control purposes.

The described system can be used e.g. for treating, diagnosing, monitoring and/or preventing signs and/or symptoms associated to pathological conditions. The electrical connection between electrodes interconnects or pads 400 and one or more external electrical and/or electronic device 500 can be done in any suitable way known in the art. With reference for instance to Figure 5, in one embodiment of the present disclosure some connection wires extend from the interconnects or pads 400 to the one or more external device 500, where e.g. an electrical pad electrically couples said wires to said device 500.Another aspect of the invention pertains a microfabrication method of the device as disclosed. With reference to Figures 9A to 9C, the method according to this disclosure is a method of manufacturing a device comprising a flexible and/or elastic substrate having at least a first and a second surface and a plurality of glassy carbon microneedles located on said first surface, said method comprising the steps of:

Patterning a plurality of polymeric pillars 15 on a first carrier support 10;

Carbonizing via pyrolysis said plurality of polymeric pillars 15 to obtain a plurality of glassy carbon microneedles 200/210/220;

Detaching the plurality of glassy carbon microneedles 200/210/220 from the first carrier support 10 by firstly penetrating said microneedles into a penetrable stamp 17, and secondly flipping the penetrable stamp 17 comprising the microneedles 200/210/220;

Transferring the penetrable stamp 17 comprising the plurality of glassy carbon microneedles 200/210/220 onto a flexible and/or elastic substrate 100, wherein said microneedles 200/210/220 are positioned onto a first surface 101 of the flexible and/or elastic substrate 100; and

Removing the penetrable stamp 17.

As a way of example, and without limitation, in Figures 9A to 9C there is depicted one embodiment of the method according to the present disclosure. For the sake of clarity, Figures 9A to 9C depict hollow microneedles 210 for simplicity and visual intelligibility only; as it will be evident, the reference to hollow microneedles 210 is not limiting in any way, and the precise example described hereinafter can be applicable to any kind of microneedle array 200 according to the invention. Accordingly, in Figures 9A to 9C it has been used reference number 210 (indicating hollow microneedles of the embodiment in these figures) but also reference number 200, to denote the microneedles might be not-hollow (such as those indicated with reference number 200 and 220 in Figure 1b). Further, and for the sake of clarity, the following example and Figures 9A to 9C make reference in more details to a particularly advantageous, but not limiting, embodiment of the invention that foresees the use of photolithographic processing for patterning microneedles arrays, said microneedles being created by pyrolysis of photoresist polymeric pillars. However, of note, glassy carbon structures can be created from the pyrolysis of polymer precursors that might not be photo patternable such as Polyacrylonitrile (PAN), Perfluoroalkoxy alkane (PAF) or other resins, but which might be for instance 3D printed.

During a first method step, a so-called temporary substrate 10 is provided (a carrier 10). The temporary substrate 10 acts as a physical carrier support to the elements of the device to be manufactured, and is further used for instance to align and hold-in-place the various elements of the device. The temporary substrate 10 can be for instance a silica, silicon or sapphire wafer, which are particularly suitable if a photolithography microstructuring process is put in place.

According to an embodiment of the method, the temporary substrate 10 can be coated with a (removable) layer 11, acting as a sacrificial release film. Said layer 11 can be made for instance of metals or metal oxides, such as MgO or Al2O3. Most preferably, the first carrier support 10 comprises a thin-film metal oxide sacrificial release layer 11. This approach is particularly advantageous in the frame of the present method; in fact, the initial idea of the inventors was to etch away the oxide sacrificial layer using an acidic solvent. However, unexpectedly thin oxide layers get fractured during the carbonization, thus facilitating releasing of glassy carbon three-dimensional microstructures without the need of immersing the carrier support 10 into an acid. The releasing step (using a permeable soft stamp) will be described later along this specification in much more detail.

In a subsequent step, a plurality of polymeric pillars 150 are patterned on the first carrier support 10. In Figure 9e, reference number 150 indicates the pillars 150 and reference number 15 indicates the hole therethrough. The pillars 150 represent the precursors of the microneedles of the device 1 as obtained at the end of the method, in particular the stems 202 thereof (Figure 9C), and they are therefore patterned and designed according to the needs and circumstances, in particular to the final microneedles to be included in the device. The manufacturing of the pillars 150 is not limiting, and is performed according to a method depending on the needs. Suitable patterning techniques may include 3D printing, moulding or photolithography, as anticipated.

Using photolithography as a prototypical, non-limiting embodiment for a manufacturing process, the base pad 13 of the pillars 150 is therefore patterned by coating a first photoresist polymeric layer 12, for instance a 100 µm layer of SU-8, on top of the carrier support 10, and exposing to UV light said photoresist polymeric layer 12 to obtain the base pads 13 of pillar arrays 150. The base pads 13 are precursors of the support bases 203 (Figure 9C) of the device. This step is performed, for negative photoresists such as SU-8, through a topside photoresist exposure to suitable UV light. The next step is to coat at least one additional photoresist polymeric layer 14 (for instance of 300 µm) but typically several layers 14 one on top of the others until a suitable thickness is reached, in which the body of the pillars 150 will be patterned. The coated thickness will define the length of the pillars, and therefore of the subsequent obtainable microneedles. In implemented embodiments, a photoresist polymeric layer 14 is obtained by coating twice 300 µm of a photoresist (e.g. SU-8) to reach a 600µm thickness.

This coating step is preferably preceded by preparation step (not shown) of surface stripping with O2 plasma or dehydration on a 200°C hotplate. Suitable coating procedure may be spin-coating as known in the art. After the spin-coating, the wafer 10 is soft baked to evaporate solvents and the resin for the following exposition step.

UV exposure is the crucial step where the shape is tried to be obtained through the backside exposure technique (for negative photoresist polymers) and by varying some parameters of the mask aligner. As the pillars 150 are obtained through backside exposure, glass wafers 10 (the carrier 10 is made of glass) are preferably used as they allow the light to pass through the transparent substrate from below.

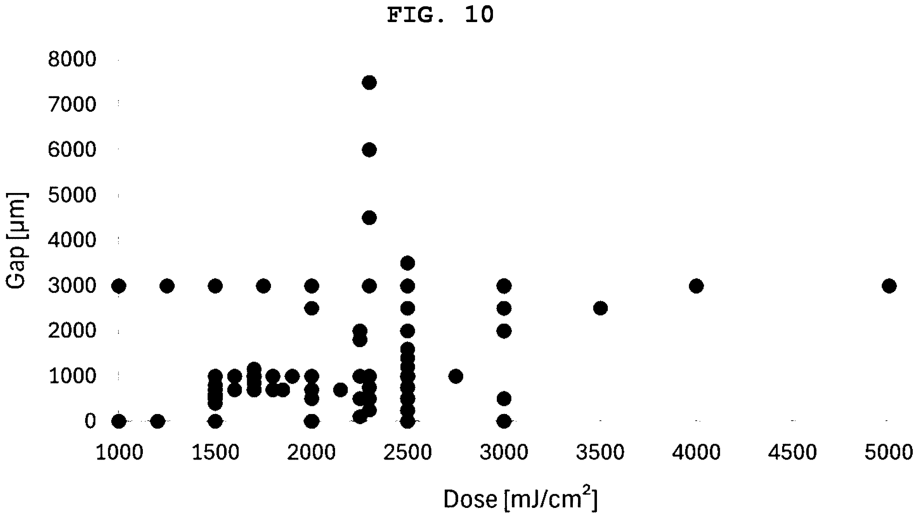

to obtain tapered or spiky structures for the pillars 150 (and therefore for the microneedles), the most critical step of the process flow to obtain these structures happens during the UV exposure through a technique called backside exposure (BSE), performed on a mask aligner and in the frame of the use of negative photoresist polymers (e.g. SU-8). The glass wafer 10 is flipped upside down, with the photoresist 14 facing down to a mask aligner chuck, and the glass substrate facing up, between the light source and the polymeric resin. This way, due to the diffusion and diffraction of light and the negative nature of SU-8, the cross-linking will be stronger at the base (on the substrate side) than the tip of the pillars. In order to influence the taper angle of these structures (in case tapered or even spiked microneedles are sought, having a base and a narrower first end), two parameters can be adjusted on the mask aligner to perform the UV exposure step: the gap and the dose. The gap is the distance between the patterning mask and the glass substrate 10. The dose represents the amount of energy per unit area delivered to the resin 14 during the exposure. It is a product of the intensity of the UV light and the exposure time. Adjusting the dose affects how deeply the UV light penetrates the resin 14, thereby influencing the degree of crosslinking throughout the thickness of the resin layer 14, while the gap determines the uniformity and focus of the UV light on the resin surface; together, these parameters control the shape and tapering of the resulting microstructures. Figure 10 shows some real combination of dose and gap tested for the manufacturing of microneedle arrays, using SU-8 polymeric coatings ranging from 300 to 800 mm, doses ranging from 1000 to 5000 mJ/cm2 and gaps ranging from 0 to 7500 mm (UV wavelength at 365 nm).

After being exposed to UV light, the photoresin 14 still need an additional bake onto a hotplate, called post-exposure bake (PEB) to evaporate the remaining solvent and obtain stiff and rigid structures. These bakes (soft bake and PEB) should have varying temperatures and durations depending on the polymeric resin type and thickness. For example, soft bake and PEB may last together between 2 and 7 hours at a temperature comprised between 40 and 130 °C.

Finally, after PEB, the wafers are developed. For this step, they are dipped into an organic solvent solution such as propylene glycol methyl ether acetate (PGMEA, for example for 2 hours for a 600 µm SU-8 film) and then rinsed into. At the end of this step, fully developed pillars 150 are formed, and ready for the following steps to make the microneedles of the device.

Accordingly, the next step in the process consists in carbonizing via pyrolysis said plurality of polymeric pillars 150 (and their base pad 13) to obtain the plurality of glassy carbon microneedles 200/210/220. Pyrolysis takes place by placing the device in a furnace within a temperature range comprised between 800 and 1000 °C in an inert atmosphere (for instance in vacuum or in a nitrogen gas atmosphere), where polymeric pillars 15 shrink in size of 30 to 50% - the glassy carbon microneedles 200/210/220 are therefore much smaller in size than their pillars 150 precursors.

In preferred embodiments, the method comprises patterning a polymeric support in physical connection with the plurality of polymeric pillars 150, thereby forming a monolithic unit comprising the polymeric support and an array of pillars 150 and, after carbonization, a monolithic glassy carbon unit 231 comprising a support 230 and an array of microneedles 200/210/220. In one embodiment, the support 230 is formed by the support bases 203 of the microneedles 200. In another embodiment, the support 203 is derived by a precursor deposited separately (before) on the carrier 10 with respect to the pillars 150 and their base pads 13.

In a next step, the microfabrication method of the invention foresees detaching the plurality of glassy carbon microneedles 200/210/220 from the first carrier support 10 by firstly penetrating (inserting) said microneedles into a penetrable stamp 17, and secondly flipping the penetrable stamp 17 comprising the microneedles 200/210/220. This step can be subsequently followed by an optional step of at least partially coating with an electrically conductive film 18 the plurality of microneedles 200/210/220 or, where applicable, the monolithic glassy carbon unit 231 comprising a support 230 and an array of microneedles 200/210/220. The electrically conductive film 18 can be for instance a metallic film. This step has the advantage of facilitating the electrical connection with electrically conductive tracks or pads 400 located on the substrate 100, as will be shown later.

The detaching step is one of the added values in the frame of the present invention for what concerns inventiveness of the manufacturing method of the device. This technique is usually called transfer printing but, as adopted in the present application, it is different because transfer printing as commonly known does not involve the penetration of any stamp but rather just the adhesion to it. Using of soft stamp 17 (elastomeric or malleable substance) that gets penetrated by glassy carbon three-dimensional microstructures so as to keep these latter in place after their release, and being able to manipulate them and move them to the new soft substrate 100 without damage, was one of the more elegant approaches used by the inventors to overcome the trickiest problem in the manufacturing of tiny, high aspect ratio, rigid microstructures, especially because it was not obvious that glassy carbon three-dimensional microstructures would penetrate the soft stamp without breaking or that they would be held on the stamp thanks to this penetration.

The last steps of the method foresee transferring the penetrable stamp 17 comprising the plurality of glassy carbon microneedles 200/210/220 onto a flexible and/or elastic substrate 100, wherein said microneedles 200/210/220 are positioned onto a first surface 101 of the flexible and/or elastic substrate 100; and
Removing the penetrable stamp 17.

The penetrable stamp 17 allows to easily manipulate the microfabricated glassy carbon unit(s) so that this can be flipped and operatively connected to the substrate 100. As it will be evident, in the presence of tapered or spiky microneedles, the narrower (or sharp) end of the microneedles is the one stuck into the stamp 17, whereas the larger section portion of the needles (i.e., the base thereof) is the one that is put in physical (and possibly fluidic) contact with the substrate 100.

Ideally, an external stimulus or treatment should be applied to the microneedles 200/210/220 for them to keep attached to the substrate 100 and removed from the temporary stamp 17. This treatment can be pressure (the force and/or speed at which the microneedle array is pressed against the substrate 100) and/or heat (e.g. to solder a metal) and/or the application of a conductive paste so that the microneedle array is somehow glued to the substrate 100 and can be released from the stamp 17.

Use of metal coating (for example Ti/Pt/Au films) on the backside of the glassy carbon structures may facilitate, in embodiments where this is sought, the adhesion and connection of the said glassy carbon structures via soldering to electrically conductive tracks (e.g. metal contact pads) on the flexible and/or elastic substrate. This is an approach that it is possible thanks to the transfer step: after picking up the glassy carbon structures with the soft stamp 17, the newly formed glassy carbon microneedles 200/210/220 or unit 231 can be flipped and coated (e.g. sputtered) with metal films in the cleanroom.

One exemplary and non-limiting embodiment of the method according to the invention is described hereinafter. The inventors have fabricated glassy carbon microneedle arrays 200 on flexible polyimide printed circuit board 100. The microneedles are hollow cylinders 210 of 200 mm in height (h), 60 mm in outer width (d) and 30 mm in inner width. Each array consists of 3x3 microneedles (separated by a 250 mm gap, g) sitting on a common square base support 230 of 1 mm2 and 70 mm in thickness. Glassy carbon is obtained by the pyrolysis at 900 °C of SU8 photoresist 14 (previously coated, baked and exposed) sitting on a sacrificial release layer 11 of MgO (50 nm) on top of a transparent fused silica 4-inch wafer 10.

After pyrolysis, the microneedle arrays 200 (as a glassy carbon unit 231) are released from the wafer 10 attaching them to a wafer-size elastomeric stamp 17 (Ecoflex). The stamp 17 is then flipped and sputtered to coat the back of the microneedle arrays bases with a thin film (100 nm) metallic (Ti/Pt/Au) layer 18. As soft substrate, a hexagonal flexible polyimide printed circuit board 100 (PCBway) with 15 gold plated contact pads 400, covered with solder paste, was used. The stamp 17 holding the glassy carbon unit 231 is then placed on top of the flexible substrate 100 so that the microneedle arrays 200 (the support base 203) are aligned with the contact pads 400. They are pressed against the flexible substrate 100 to be released from the stamp 17 and heat is applied trough a hotplate at 130 °C to solder the glassy carbon microneedle arrays 200 to the metallic contact pads 400. The substrate 100 comprises a microfluidic circuit 300 that is operatively (physically and fluidically) connected to the channels 212 of the microneedles 200.

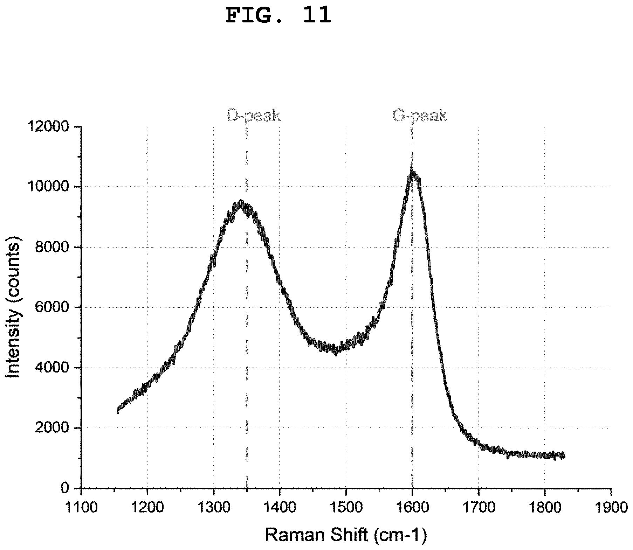

Extensive characterization of the obtained glassy carbon microneedles device has been carried out by the present inventors (see Figures 11 to 16):
Raman spectroscopy (Figure 11): This measurement shows the different molecular species present in obtained glassy carbon samples. The intensity peak on the D-band (1350 cm-1) is associated to the presence of defects and disorder within the carbon structure whereas the intensity peak on the G-band (1590 cm-1) corresponds to the presence of sp2 hybridized carbon atoms, which relates to the presence of ordered graphitic carbon. The ratio of this intensity peaks should be closed to 1 for glassy carbon samples, and that is what it is observed from this measurement.

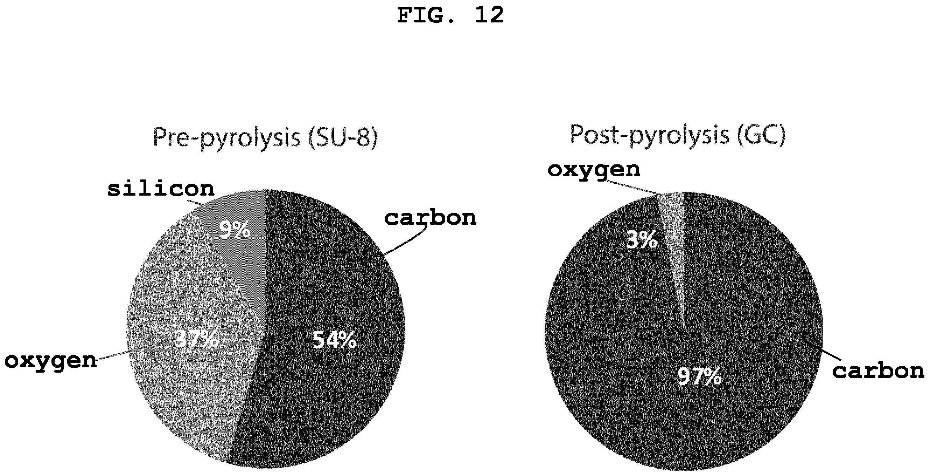

EDX spectroscopy (Figure 12): This technique allows to get quantitative information about the concentration of each chemical element present in the sample surface measuring the intensity of the characteristic X-ray peaks. inventors performed a measurement of samples before pyrolysis (they are still SU-8) and after pyrolysis (they become glassy carbon). It was observed that the concentration of carbon increases dramatically after pyrolysis, which proves the purity of glassy carbon.

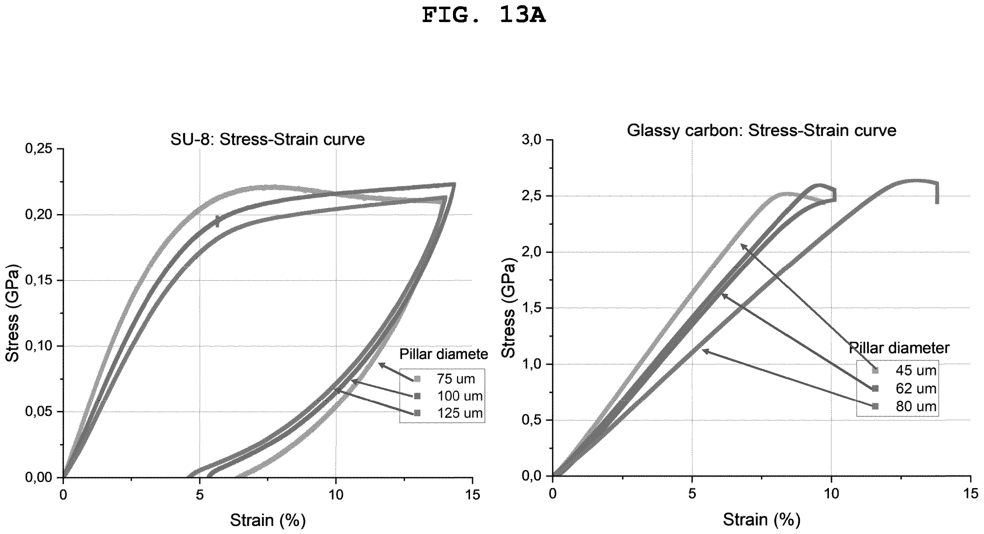

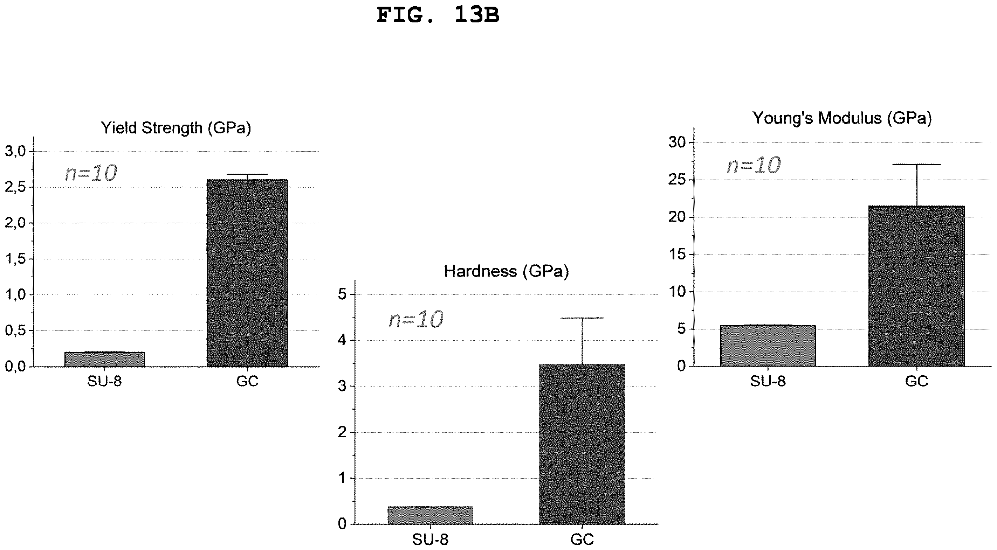

Mechanical tests (Figure 13A and 13B): Microneedles of varying diameters were mechanically tested under axial load (compressive stress) before pyrolysis and after pyrolysis to see the difference between SU-8 and GC mechanical properties. The stress-strain curve shows the elastic behaviour of SU-8 given that microneedle do not break but their maximal yield strength is around 0.25 GPa, which is not enough to effectively penetrate the skin. The stress-strain curve of GC, on the other hand, show a harder and stiffer behaviour resulting in a yield strength of 2.5 GPa after which the microneedles end up breaking. Therefore, the carbonization of the microneedles multiplies by 10 the strength of the material.

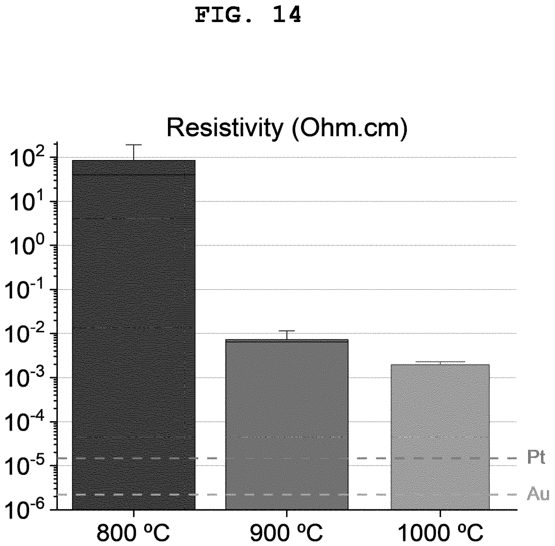

Resistivity (Figure 14): This electrical property is obtained using a 4-point probe resistance measurement on glassy carbon samples. It proves that glassy carbon is an electrically conductive material although worse than noble metals such as gold and platinum. The inventors observed that the resistivity can be reduced by increasing the maximal pyrolysis temperature (because the number of graphitic structure increases).

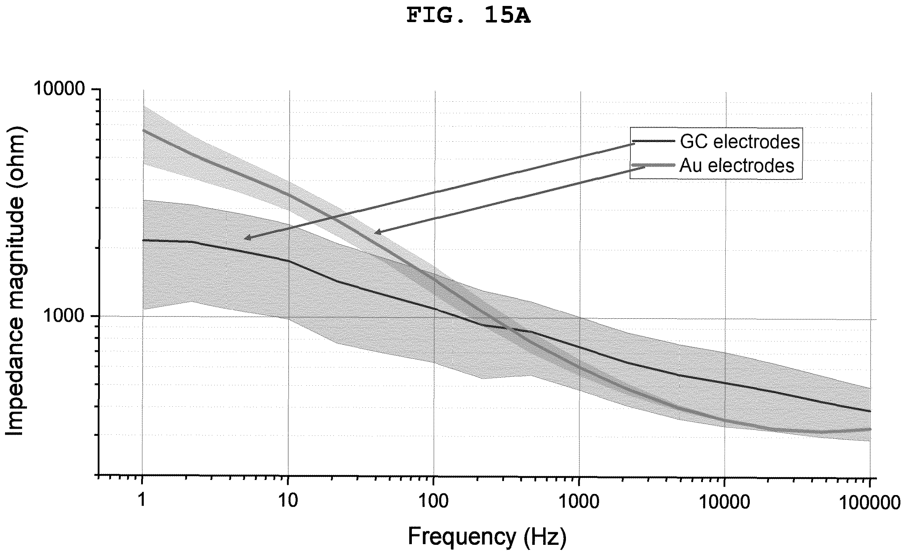

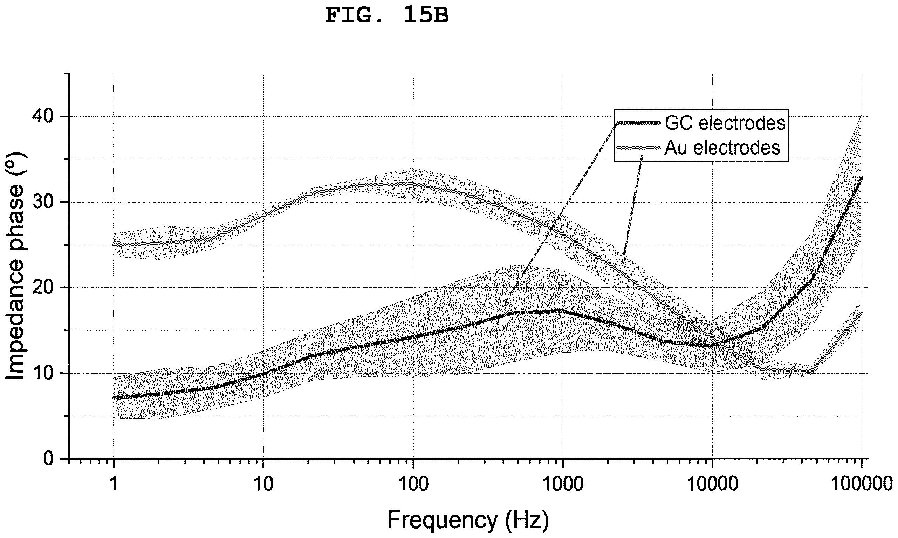

Electrochemical impedance spectroscopy (Figure 15A and 15B): This measurement is obtained immersing glassy carbon electrodes and standard gold electrodes of the same size in PBS solution in order to assess its electrochemical behaviours at different frequencies. We observe that glassy carbon electrodes are more resistive than gold electrodes (impedance phase) but their impedance magnitude is in the same order of magnitude.

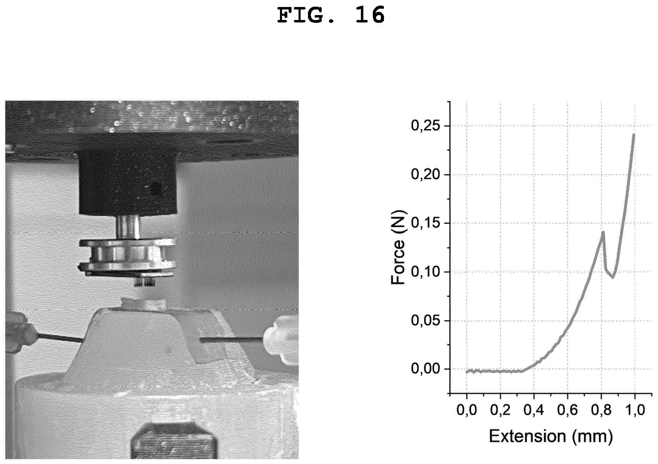

Skin penetration proof (Figure 16): The axial force is measured while penetrating animal skin (from pig inner ear) with glassy carbon microneedles. The drop in the force is proof of effective penetration of the first skin layer, the epidermis, without microneedle fracture.

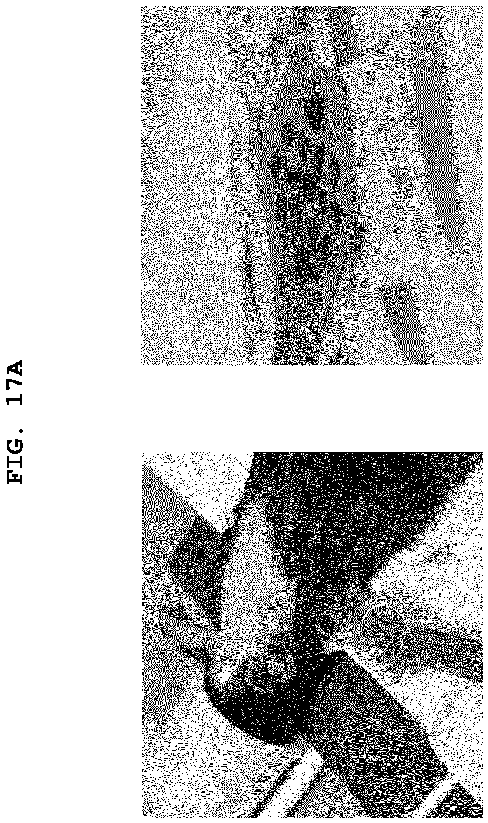

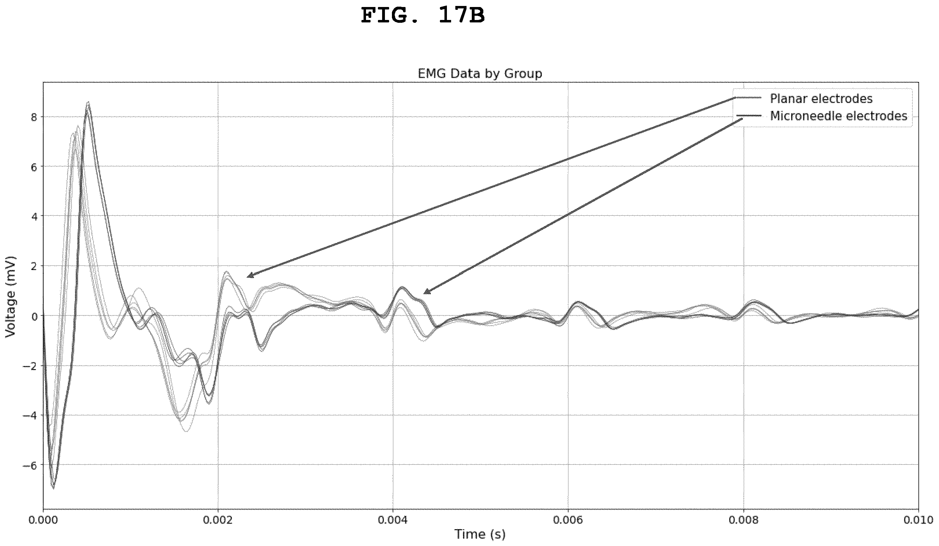

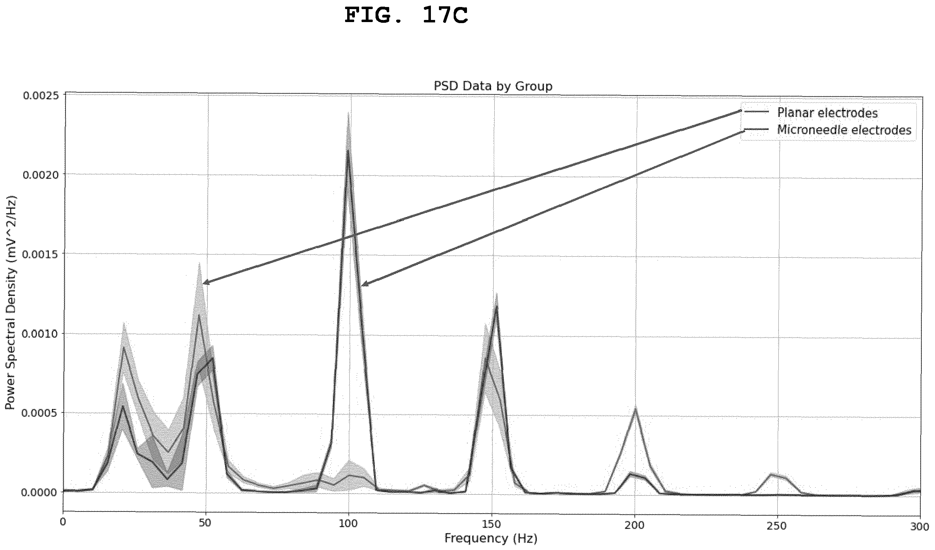

As shown in Figures 17A to 17C, the inventors further fabricated a device containing microneedle array electrodes as well as planar electrodes of the same material (glassy carbon) in order to compare their properties when recording electrophysiological data from animals. A first attempt was tried on mouse skin, whose hair was removed using depilatory cream. The back of the mouse was stimulated using copper electrodes and a current stimulator delivering 1mA 40Hz signal. The device was placed on the arm of the mouse and was held with medical tape while the muscle activity was being recorded. After the experiment, the device was removed and none of the microneedles had broken upon insertion and removal.

The microneedle electrodes appear to offer superior performance compared to planar electrodes for recording EMG signals. They provide higher amplitude signals, better fidelity, less variability, and a more stable baseline, which are all crucial for accurate and reliable EMG measurements. These advantages likely stem from the ability of microneedle electrodes to penetrate the skin and establish closer contact with the muscle tissue, reducing the impact of skin impedance and other artifacts that can affect planar electrode recording.

One aspect of the invention relates to the use of the device or system of the invention as a bidirectional stimulation and/or sensing bioelectronic interface for wearable implementation of haptic feedback on humans, including tactile or thermal sensation encoding in virtual- or augmented-reality environments or for teleoperation purposes. Haptic feedback devices using electrical or fluidic stimulation of the human skin already exist and are known in the art, such as for instance devices described in Tezuka, M., Kitamura, N. & Miki, N. Information transfer using wearable thin electrotactile displays with microneedle electrodes. Jpn. J. Appl. Phys. 55, 06GP15 (2016) https://doi.org/10.7567/JJAP.55.06GP15 and Lu, J., Liu, Z., Brooks, J. & Lopes, P. Chemical Haptics: Rendering Haptic Sensations via Topical Stimulants. (ACM 34th UIST Symposium, Virtual Event USA, 2021). https://doi.org/10.1145/3472749.3474747. However, up to the inventor's knowledge, none of them include glassy carbon microneedle arrays for applications in haptics exploiting at a time electrical, fluidic and/or electrofluidic Compared to the state-of-the-art the invention presents a robust skin interface able of providing with reliable multimodal stimulation.

As it will be evident to a person skilled in the art, still another aspect of the invention relates to the use of the device or system of the invention as a bidirectional stimulation and/or sensing bioelectronic interface for diagnostic or therapeutic applications including transdermal delivery of compounds on a subject, biochemical sensing of biological markers in a subject, electrophysiological monitoring and/or transdermal/organ electrical stimulation in a subject.

The device of the invention is intended to be used in one embodiment as a peripheral or Central Nervous System (CNS) interface, acting as a bridge between the peripheral/central nervous system and external devices to bidirectionally transducing recorded information and/or sending signals between the human body and a machine processor, and particularly as a fixed or removable implant configured to interface with a biological surface of a subject with the purpose of electrically sensing and/or stimulating an electrical, fluidic or electrofluidic activity in said subject. Accordingly, the implantable electrical/electronic/electrofluidic biomedical device of the invention can be configured as a way of example as a fixed or removable neural or nerve implant, heart implant, kidney implant, pancreatic implant, bladder implant, retina implant, skin implant or gut implant, to mention some.

Accordingly, the method foresees implanting an electrical or electronic biomedical device according to the present disclosure on a biological surface located on a portion of the brain, the heart, the gut, the pancreas, the bladder, a blood vessel, the skin, a kidney and/or an eye of said subject in a relatively non-invasive manner.

In view of what said above up to now, it would be evident for a person skilled in the art that in certain embodiments the device of the invention can be used for electrically sensing a physiological parameter or stimulating an electrical response in a biological surface, such as the brain or the skin, of a subject. The sensing or stimulation activity can be performed in the frame of a therapeutic or preventive (e.g. for diagnostic purposes) medical treatment in a subject. Accordingly, a further aspect of the invention concerns the use of a device or of a system according to the present disclosure for sensing a physiological signal and/or stimulating an electrical activity and/or pharmacological activity of a biological surface, such as the brain or the skin, of a subject. Advantageously, the use of the device or of the system can be intended for treating one or more pathological conditions. For instance, the device, system and methods according to the present disclosure can be used in a variety of situations for treating one or more pathological conditions wherein sensing and/or electrically or electrofluidically stimulating a biological surface of a subject might be beneficial, and in applications related to peripheral nerve pathological conditions including sensory feedback and/or motor feedback.

As a way of example, when in use, the electrode structure 300 is electrically connected through conductors 400 to an external device 500 for delivering an electrical current and/or for biological signal sensing, such as a neuromuscular electrical stimulator or a biological signal acquisition system (e.g. that can acquire for example electromyography -EMG- signals), depending on the application of interest.

The device and system according to the present disclosure can be used in a variety of situations for treating one or more pathological conditions. A non-limiting, exemplary list of uses of the biomedical device of the present invention comprises retinal, subretinal, and suprachoroidal implants or prosthesis for the treatment of degenerative retinal diseases such as retinitis pigmentosa or age-related macular degeneration (AMD); wound healing, for instance for diabetic patients; delivery of compounds for cosmetics; pain relief for neuropathy patients; phrenic nerve stimulators and diaphragm pacemakers to restore breathing function in patients with breathing disorders such as central hypoventilation syndrome (CCHS), central sleep apnea, and diaphragm paralysis; cochlear implants or auditory brainstem implants for the treatment of partial or profound deafness resulting from inner ear damage; stimulation of the brainstem for the treatment of tinnitus; stimulation of the brain for recovery in stroke patients or to treat migraines; electrocorticography (ECoG) to record electrical activity from the cerebral cortex; pacemakers and implantable cardioverter defibrillators; electrical recording for epicardial and endocardial mapping; bladder implants for the treatment of bladder disorders such as detrusor hyperreflexia, detrusor areflexia, overactive bladder syndrome, and urine retention; electrical stimulation of the sphincter for constipation; electrical stimulators for the therapy of pain relief and management thereof, for Parkinson's disease, dystonia or epilepsy; peripheral nerve stimulators for e.g. stimulation of the common peroneal nerve to provide dorsiflexion of the foot and restore muscle movements; lower esophagus stimulators for preventing or treating gastroesophageal reflux; vagal blocking devices for the treatment of obesity by suppressing the sensation of hunger; neuronal interfaces for treating spinal cord injury, strokes or degenerative disorders such as amyotrophic lateral sclerosis cerebral palsy, muscular dystrophy and chronic neuropathic pain, and the like.

The entire system may of course, and preferably, comprise a data processing apparatus 600 operatively connected with the system, the data processing apparatus having a processor comprising instructions configured to operate the system to perform a method of using the device of the invention depending on the needs. The data processing apparatus 600 of the invention can comprise any suitable device such as computers, smartphones, tablets, voice-activated devices (i.e. smart speakers/voice assistants) and the like.

In one embodiment, the data processing apparatus 600 comprises memory storing software modules that provide functionality when executed by the processor. The modules include an operating system that provides operating system functionality for the apparatus. The system, in embodiments that transmit and/or receive data from remote sources, may further include a communication device, such as a network interface card, to provide mobile wireless communication, such as Bluetooth, infrared, radio, Wi-Fi, cellular network, or other next-generation wireless-data network communication. In other embodiments, communication device provides a wired network connection, such as an Ethernet connection or a modem.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention is not limited to the described embodiments, and should be given the broadest reasonable interpretation in accordance with the language of the appended claims.

## Claims

1. A device (1) for interfacing a bodily tissue comprising:
a) a flexible and/or elastic substrate (100) including at least a first surface (101) and a second surface (102) and
b) at least one microneedle (200) including a body on at least one of said first surface (101) and second surface (102) of the flexible and/or elastic substrate (100), **characterized in that** the body of the at least one microneedle (200)
i) is made of glassy carbon and
ii) is operatively coupled with at least one fluidic channel (300) and/or with at least one electrically conductive track or pad (400), running in or on the flexible and/or elastic substrate (100).

2. The device (1) according to claim 1, wherein the body is operatively coupled with the at least one fluidic channel (300), the body is at least in part hollow, the hollow body part is in fluid communication with the at least one fluidic channel (300) for operatively conduct a fluid from/to the fluidic channel (300) to/from an aperture of the hollow body part, and the body is in electric contact with said at least one electrically conductive track or pad (400).

3. The device (1) according to claim 1, wherein the body is operatively coupled with the at least one electrically conductive track or pad (400), the body is in electric contact with said at least one electrically conductive track or pad (400), and the body is non-hollow.

4. The device according to claim 2, wherein the body includes a stem (202) and a support base (203) of the stem (203) extending from a portion of the stem (202) proximal to the flexible and/or elastic substrate (100), wherein the flexible and/or elastic substrate (100) includes an aperture (A) through a portion of said first surface (101) in communication with the fluidic channel (300), wherein the support base (203) of the stem (202) is arranged on the first surface (101) around the aperture (A) to determine a fluid path for the fluid from/to the fluidic channel (300) to/from the stem (203).

5. The device (1) according to claim 4, wherein the at least one microneedle (200) protrudes from a glassy carbon support (230), monolithic with the at least one microneedle (200), wherein the glassy carbon support (230) is coupled to the flexible and/or elastic substrate (100) to realize said operative coupling between the at least one microneedle (200) and the at least one fluidic channel (300) and/or with at least one electrically conductive track or pad (400), wherein the glassy carbon support (230) includes the support base (203) of the stem (202).

6. The device according to any previous claim, wherein the at least one microneedle (200) is at least partially coated with an electrically conductive film.

7. The device according to any previous claim, wherein said at least one microneedle (200) includes a plurality of microneedles (200) arranged according to a predetermined pattern.

8. The device according to any previous claim, wherein the flexible and/or elastic substrate comprises a flexible printed circuit board (PCB).

9. A microfabrication method for manufacturing a device for interfacing a bodily tissue, said method comprising the steps of:
a) preparing a flexible and/or elastic substrate (100) having at least a first surface (101) and a second surface (102), said step of preparing the flexible and/or elastic substrate (100) including forming at least one fluidic channel (300) and/or at least one electrically conductive track or pad (400) in or on the flexible and/or elastic substrate (100);
b) depositing at least one polymeric body (150) on a carrier (10), said polymeric body (150) having the shape of a needle and protruding from a proximal end on the carrier (10) to a distal end distanced from 10 to 2000 microns from the carrier (10);
c) Carbonizing via pyrolysis the at least one polymeric body (150) and obtaining a corresponding at least one glassy carbon microneedle (200);
d) Detaching the at least one microneedle (200) from the carrier (10);
e) Transferring the at least one microneedle (200) onto the flexible and/or elastic substrate (100), to operatively coupling the at least one microneedle (200) with the at least one fluidic channel (300) and/or with the at least one electrically conductive track or pad (400).

10. The microfabrication method of claim 9 wherein
said step d) of detaching from the carrier (10) includes inserting a stem (202) of the at least one microneedle (200) into a penetrable stamp (17), wherein detaching from the carrier (10) is made by flipping the stamp (17) with the at least one microneedle (200); and wherein
said step e) of transferring the at least one microneedle (200) includes transferring the stamp (17) with the at least one microneedle (200) inserted therein onto the flexible and/or elastic substrate (100) and wherein the method further comprises
a step f) of removing the stamp (17) from the at least one microneedle (200) after transferring.

11. The microfabrication method of claim 10, wherein said step b) of providing at least one polymeric body (150) on the carrier comprises patterning a polymeric support (230) on the carrier (10) and patterning the polymeric body (150) on the polymeric support (230), wherein said step c) of carbonizing carbonizes the at least one polymeric body (150) into the stem (202) of the microneedle (200) and carbonizing the polymeric support (230) into a support base (203) of the stem, said stem (202) and support base (203) being part of or forming a monolithic glassy carbon unit.

12. The microfabrication method of claims 9 or 10, further comprising a step of at least partially coating the at least one microneedle with an electrically conductive film, preferably with a metallic film, said step of partially coating being carried before detaching the at least one microneedle (200) from the carrier.

13. The microfabrication method of claim 9, wherein the carrier comprises a thin-film oxide sacrificial release layer, and the at least one polymeric body (15) in said step b) is provided on the thin-film oxide sacrificial release layer of the stamp.

14. The microfabrication method of claim 9, wherein said step e) of transferring the at least one microneedle (200) includes electrically connecting said at least one electrically conductive track or pad to the at least one microneedle (200).

15. Use of the device for interfacing a bodily tissue according to claims 1 to 8 wherein the device (1) is worn on a body portion with the flexible and/or elastic substrate (100) adhering to the body portion and the at least one microneedle (200) partially entering the bodily tissue to transfer at least one among fluid, electric current and heat between the device and the bodily tissue, , wherein said transfer of fluid, current and heat is suitable to implement at least one among a virtual- or augmented-reality haptic application, a transdermal delivery of the fluid, a biochemical sensing of biological markers, an electrophysiological monitoring and/or a transdermal/organ electrical stimulation.
